# EUROPEAN PATENT APPLICATION

(11) **EP 4 596 010 A1**
(43) Date of publication of application: **06.08.2025**
(21) Application number: 23870113.0
(22) Date of filing: 29.08.2023
(51) Int. Cl.: A61M 16/00, A61B 5/0205

(54) **VENTILATION DEVICE HOST, VENTILATION DEVICE, AND MEDICAL DEVICE**

(30) Priority: 27.09.2022 CN 202211185805; 05.05.2023 WO PCT/CN2023/092375
(71) Applicant: Shenzhen Mindray Bio-Medical Electronics Co., Ltd., Shenzhen, Guangdong 518057 (CN)
(72) Inventor: HOU, Huan, Shenzhen, Guangdong 518057 (CN); LAI, Deng Shui, Shenzhen, Guangdong 518057 (CN); WEI, Kai, Shenzhen, Guangdong 518057 (CN); WANG, Hao, Shenzhen, Guangdong 518057 (CN)
(74) Representative: KIPA AB
(86) International application number: PCT/CN2023/115602
(87) International publication number: WO 2024/066872

(57) **Abstract**

Disclosed are a ventilation device host (100), a ventilation device, and a medical device (1000), wherein the ventilation device host (100) comprises a housing (10), a gas path assembly (20), and a pressure monitoring assembly (30). The housing (10) is provided with an inner cavity (11), and an outer side of the housing (10) is recessed inwards to form an accommodating cavity (12). The gas path assembly (20) comprises an oxygen control assembly (24) and an output interface (22). The oxygen control assembly (24) is configured for connecting an oxygen supply apparatus, and the output interface (22) is configured for connecting a suction branch and delivering gas formed by mixing oxygen and air to a patient by means of the suction branch. The pressure monitoring assembly (30) is at least configured for communicating with the gas path assembly (20) to monitor relevant parameters of input and/or output gas of the gas path assembly (20). The accommodating cavity (12) is configured for detachably accommodating at least one portable medical device (200). The portable medical device (200) comprises an interface side (201); the interface side (201) of the portable medical device (200) is provided with a cable interface (202); when the portable medical device (200) is accommodated in the accommodating cavity (12), the output interface (22) and the cable interface (202) are located on different sides of the housing (10).

## Description

### TECHNICAL FIELD

The disclosure relates to the technical field of medical device, and more particularly to a main unit of ventilation device, a ventilation device, and a medical device.

### BACKGROUND

Existing ventilation device usually only serves for assisting respiration, and has relatively simple functions which cannot be expanded. In some emergency situations, medical staff need to carry not only ventilation device but also other medical device, such as monitoring instruments. The large number of medical devices are troublesome to wire and transport, which is not conducive to the first aid of patients.

### SUMMARY

In view of this, this disclosure proposes a main unit of ventilation device, a ventilation device and a medical device.

According to a first aspect, an embodiment of this disclosure provides a main unit of ventilation device including:
a housing, which is provided with an inner cavity, wherein an outer side of the housing is recessed inward to form an accommodation cavity;
a gas path assembly, which includes an oxygen control assembly and an output interface; wherein the oxygen control assembly is connected with an oxygen supply apparatus; the output interface is connected with an inspiratory branch, and configured to deliver to a patient, through the inspiratory branch, a gas which is formed by mixing oxygen and air;
a pressure monitoring assembly, which is arranged inside the inner cavity, wherein the pressure monitoring assembly is at least connected with the gas path assembly, so as to monitor a relevant parameter of an input gas and/or an output gas of the gas path assembly;
wherein, the accommodation cavity is configured to removably accommodate at least one portable medical device, wherein the portable medical device includes an interface side, which side is provided with a cable interface, wherein when the portable medical device is accommodated inside the accommodation cavity, the output interface and the cable interface are located on different sides of the housing.

According to a second aspect, an embodiment of this disclosure provides a main unit of ventilation device including:
a housing, which is provided with an inner cavity, wherein an outer side of the housing is recessed inward to form an accommodation cavity;
a display screen, which is assembled at the housing;
a gas path assembly, which includes an oxygen control assembly and an output interface; wherein the oxygen control assembly is connected with an oxygen supply apparatus; the output interface is connected with an inspiratory branch and deliver a gas to a patient through the inspiratory branch;
a pressure monitoring assembly, which is arranged inside the inner cavity, wherein the pressure monitoring assembly is at least connected with the gas path assembly, so as to monitor a relevant parameter of an input gas and/or an output gas of the gas path assembly;
wherein, the accommodation cavity is configured to removably accommodate at least one portable medical device, wherein the portable medical device includes an interface side, which side is provided with a cable interface, wherein when the portable medical device is accommodated inside the accommodation cavity, the output interface and the cable interface are located on two adjacent sides of the housing, and the output interface is located on a side of the housing, at which side the display screen is assembled.

According to a third aspect, an embodiment of this disclosure provides a main unit of ventilation device including:
a housing, which is provided with an inner cavity, wherein an outer side of the housing is recessed inward to form an accommodation cavity;
a gas path assembly, which is arranged inside the inner cavity, wherein the gas path assembly includes an input interface and an output interface, wherein the input interface is configured to input a gas formed by mixing oxygen and air, the output interface is connected with an inspiratory branch, and configured to deliver said gas to a patient through the inspiratory branch;
a pressure monitoring assembly, which is arranged inside the inner cavity, wherein the pressure monitoring assembly is at least connected with the gas path assembly, so as to monitor a relevant parameter of an input gas and/or an output gas of the gas path assembly;
wherein, the accommodation cavity is configured to removably accommodate at least one portable medical device, and includes an opening for inserting the portable medical device; wherein the output interface and at least a portion of the opening are located on different sides of the housing.

According to a fourth aspect, an embodiment of this disclosure provides a ventilation device including an inspiratory branch and the above-mentioned main unit of ventilation device, wherein the inspiratory branch is connected with the gas path assembly.

According to a fifth aspect, an embodiment of this disclosure provides a medical device including:
a trolley, which includes a column, and a support frame which is assembled at the column;
an infusion pump assembly, which is assembled at the column;
the above-mentioned main unit of ventilation device, which is placed on the support frame.

It can be seen from the above technical solutions that the main unit of ventilation device proposed in the first aspect of this disclosure, firstly, by arranging an accommodation cavity on an outer side of the housing, the user can assemble a portable medical device into the accommodation cavity according to an actual use requirement, so as to expand a function of the ventilation device and broaden a scope of use. In some emergency situations, it can reduce the trouble of wiring and carrying instrument. Secondly, by arranging the portable medical device to be removably accommodated inside the accommodation cavity, when the user does not need to use the extended function of the portable medical device, the portable medical device can be removed and the main unit of ventilation device can be used only as a ventilation device, thereby improving a flexibility of use and reducing an overall weight of the main unit of ventilation device. In addition, by arranging the portable medical device to be accommodated inside the accommodation cavity, and enabling a cable interface of the portable medical device and an output interface of the gas path assembly to be located on opposite sides of the housing, a connection pipeline of the gas path assembly, and a connection cable of the portable medical device are separated, which can avoid cluttered connection pipeline and facilitate the use of the main unit of ventilation device.

### BRIEF DESCRIPTION OF THE DRAWINGS

In order to more clearly explain the embodiments of this disclosure, the following briefly introduces the drawings which are needed to be used in the description of the embodiments. It is obvious that the drawings in the following description are only some embodiments of this disclosure. For those skilled in the art, other drawings can be obtained from these accompanying drawings without paying any creative works.
FIG. 1 is a structural diagram of a main unit of ventilation device from a first perspective according to an embodiment of this disclosure.
FIG. 2 is an exploded diagram of a main unit of ventilation device according to an embodiment of this disclosure.
FIG. 3 is a structural diagram of a main unit of ventilation device from a second viewing angle according to an embodiment of this disclosure.
FIG. 4 is an exploded diagram of a partial structure of a main unit of ventilation device according to an embodiment of this disclosure.
FIG. 5A is a structural diagram of a gas path assembly according to an embodiment of this disclosure.
FIG. 5B is an exploded diagram of a gas path assembly according to an embodiment of this disclosure.
FIG. 6 is a simplified diagram of a main unit of ventilation device according to another embodiment of this disclosure.
FIG. 7 is a simplified diagram of a main unit of ventilation device according to another embodiment of this disclosure.
FIG. 8 is a structural diagram of a main unit of ventilation device according to an embodiment of this disclosure.
FIG. 9 is a partially enlarged diagram of point C in FIG. 8.
FIG. 10 is a diagram of cooperation between a position-limiting member and an accommodation cavity according to another embodiment of this disclosure.
FIG. 11 is a structural diagram of a main unit of ventilation device according to an embodiment of this disclosure.
FIG. 12 is a cross-sectional diagram of a main unit of ventilation device according to an embodiment of this disclosure.
FIG. 13 is a partially enlarged diagram of point D in FIG. 12.
FIG. 14 is a structural diagram of a pushing-out mechanism according to an embodiment of this disclosure.
FIG. 15 is a diagram of a partial structure of a main unit of ventilation device according to an embodiment of this disclosure.
FIG. 16 is a structural diagram of a portable medical device according to an embodiment of this disclosure.
FIG. 17 is a cross-sectional diagram of a main unit of ventilation device according to another embodiment of this disclosure.
FIG. 18 is a partially enlarged diagram of point E in FIG. 17.
FIG. 19 is a structural diagram of a main unit of ventilation device according to an embodiment of this disclosure.
FIG. 20 is a structural diagram of a main unit of ventilation device according to an embodiment of this disclosure.
FIG. 21 is an internal structural diagram of a main unit of ventilation device according to an embodiment of this disclosure.
FIG. 22 is an exploded internal structural diagram of a main unit of ventilation device according to an embodiment of this disclosure.
FIG. 23 is an exploded diagram of a partial structure of a main unit of ventilation device according to an embodiment of this disclosure.
FIG. 24 is an exploded structural diagram of a main unit of ventilation device according to an embodiment of this disclosure.
FIG. 25 is a diagram of a partial structure of a main unit of ventilation device according to an embodiment of this disclosure.
FIG. 26 is a structural diagram of a main unit of ventilation device according to another embodiment of this disclosure.
FIG. 27 is a structural diagram of a medical device according to an embodiment of this disclosure.

### DESCRIPTION OF THE PREFERRED EMBODIMENTS

The technical solutions in the embodiments of this disclosure are described clearly and completely below in combination with the drawings in the embodiments of this disclosure. Obviously, the described embodiments are just some of the embodiments of this disclosure, not all of them. According to the embodiments in this disclosure, all other embodiments obtained by those skilled in the art without making creative work fall within the protection scope of this disclosure.

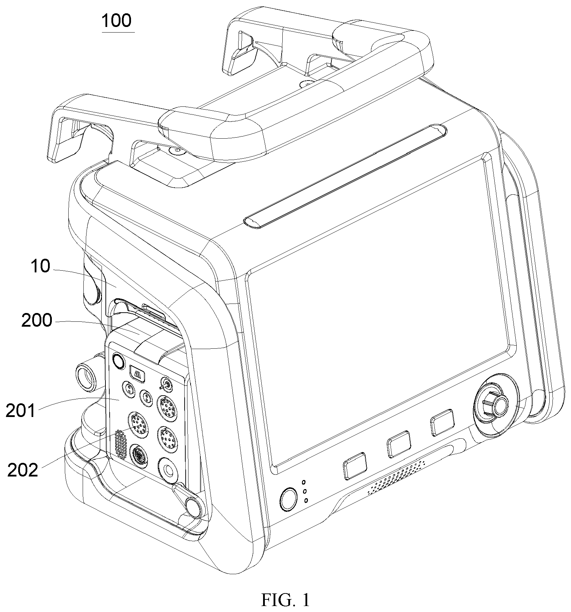

As shown in FIGS. 1 to 5B, an embodiment of this disclosure proposes a main unit of ventilation device 100, which can be but is not limited to a main unit of ventilator. The proposed main unit of ventilation device 100 includes a housing 10, a gas path assembly 20 and a pressure monitoring assembly 30. The housing 10 is provided with an inner cavity 11, and an outer side of the housing 10 is recessed inward to form an accommodation cavity 12. The gas path assembly 20 includes an oxygen control assembly 24 and an output interface 22. The oxygen control assembly 24 is connected with an oxygen supply apparatus, and the output interface 22 is connected with an inspiratory branch and deliver a gas formed by mixing oxygen and air to a patient through the inspiratory branch. The pressure monitoring assembly 30 is arranged inside the inner cavity 11, and at least connected with the gas path assembly 20 to monitor a relevant parameter of an input gas and/or an output gas of the gas path assembly 20. It should be noted that the pressure monitoring assembly 30 being connected with the gas path assembly 20, can be that the pressure monitoring assembly 30 and the gas path assembly 20 are directly connected with each other, or the pressure monitoring assembly 30 and the gas path assembly 20 are indirectly connected with each other. For example, the pressure monitoring assembly 30 is connected with the inspiratory branch, and the gas path assembly 20 is also connected with the inspiratory branch, and the pressure monitoring assembly 30 and the gas path assembly 20 are connected through the inspiratory branch. The accommodation cavity 12 is configured to removably accommodate at least one portable medical device 200, wherein the portable medical device 200 includes an interface side 201, wherein the interface side 201 of the portable medical device 200 is provided with a cable interface 202, wherein when the portable medical device 200 is accommodated inside the accommodation cavity 12, the output interface 22 and the cable interface 202 are located on different sides of the housing 10. It should be noted that the output interface 22 and the cable interface 202 being located on different sides of the housing 10, includes that the output interface 22 and the cable interface 202 are located on opposite sides of the housing 10, as well as includes that the output interface 22 and the cable interface 202 are located on adjacent sides of the housing 10. The portable medical device 200 may be, but is not limited to, a monitoring device.

The main unit of ventilation device 100 proposed in the embodiment of this disclosure, firstly, by arranging an accommodation cavity 12 on an outer side of the housing 10, the user can assemble a portable medical device 200 into the accommodation cavity 12 according to an actual use requirement, so as to expand a function of the ventilation device and broaden a scope of use. In some emergency situations, it can reduce the trouble of wiring and carrying instrument. Secondly, by arranging the portable medical device 200 to be removably accommodated inside the accommodation cavity 12, when the user does not need to use the extended function of the portable medical device 200, the portable medical device 200 can be removed, and the main unit of ventilation device 100 can be used only as a ventilation device, thereby improving a flexibility of use and reducing an overall weight of the main unit of ventilation device 100. In addition, by arranging the portable medical device 200 to be accommodated inside the accommodation cavity 12, and enabling a cable interface 202 of the portable medical device 200 and an output interface 22 of the gas path assembly 20 to be located on different sides of the housing 10, a connection pipeline of the gas path assembly 20 and a connection cable of the portable medical device 200 are separated, which can avoid cluttered connection pipeline and facilitate the use of the main unit of ventilation device 100.

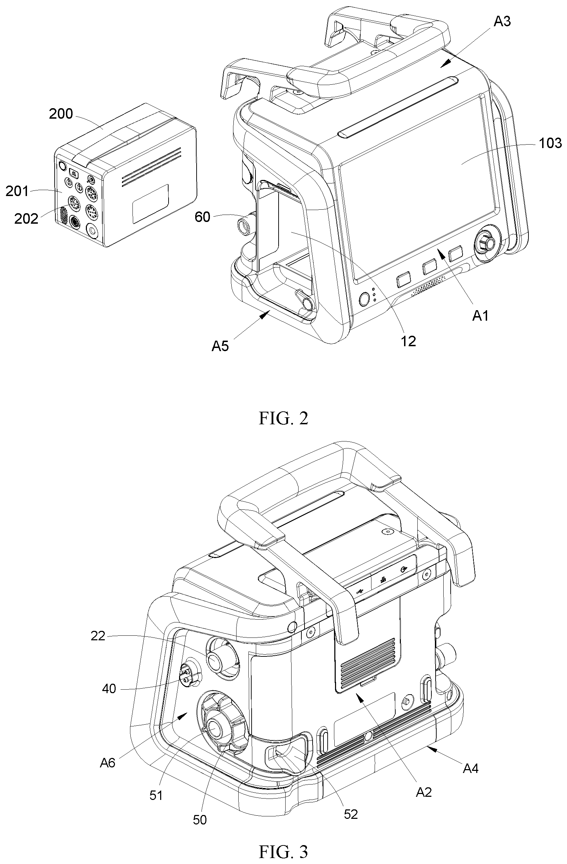

As shown in FIGS. 2 and 3, exemplarily, the housing 10 includes a front plate A1, a back plate A2, a top plate A3, a bottom plate A4, a left plate A5 and a right plate A6, the front plate A1 is arranged opposite to the back plate A2, the top plate A3 is arranged opposite to the bottom plate A4, the left plate A5 is arranged opposite to the right plate A6, one of the cable interface 202 and the output interface 22 is located on the left plate A5 of the housing 10, while the other one of the cable interface 202 and the output interface 22 is located on the right plate A6 of the housing 10. Of course, one of the cable interface 202 and the output interface 22 may be located on the back plate A2 of the housing 10, and the other one of the cable interface 202 and the output interface 22 may be located on the front plate A1 of the housing 10, which may be determined according to an actual design requirement.

It should be noted that the front plate A1, the back plate A2, the top plate A3, the bottom plate A4, the left plate A5 and the right plate A6 refer to a main unit of ventilation device 100 in normal use. The front plate A1 is a plate which faces an operator, and is generally a side with an operation keys and/or a display screen.

It should also be noted that the output interface 22 and the cable interface 202 are not limited to being located on two opposite sides of the housing 10, and the output interface 22 and the cable interface 202 can also be located on any adjacent sides of the front plate A1, the back plate A2, the top plate A3, the left plate A5 and the right plate A6, as long as the output interface 22 and the cable interface 202 are located on different sides of the housing 10, such that a connection pipeline of the gas path assembly 20, and a connection cable of the portable medical device 200 are separated, which can avoid cluttered connection pipeline and facilitate the use of the main unit of ventilation device.

In an embodiment, the cable interface 202 is located on a side where the left plate A5 is located, and the output interface 22 is located on the right plate A6.

In an embodiment, the gas path assembly 20 includes a gas output assembly 23, which is configured to receive a gas which is formed by mixing oxygen and air. The gas output assembly 23 includes the output interface 22.

As shown in FIG. 3, in an embodiment, the main unit of ventilation device 100 further includes a pressure sampling interface 40, which is connected with the pressure monitoring assembly 30 and the inspiratory branch, wherein the pressure sampling interface 40 and the output interface 22 are located on a same side of the housing 10. In this embodiment, a connection pipeline of the pressure sampling interface 40 and a connection pipeline of the gas path assembly 20 are located on the same side of the housing 10, and are separated from the connection cable of the portable medical device 200, which can avoid cluttered connection pipeline and facilitate the use of the main unit of ventilation device 100.

It should be noted that the pressure sampling interface 40 is not limited to the above-mentioned implementation mode. For example, in another embodiment, the main unit of ventilation device 10 further includes an expiratory valve 50, which is assembled at the housing 10 and configured to receive a gas exhaled by a patient. The pressure sampling interface 40 is arranged inside the housing 10, and connected with the pressure monitoring assembly 30 and the expiratory valve 50. The pressure monitoring assembly 30 is configured to monitor a relevant parameter of a gas at the expiratory valve 50.

As shown in FIG.3, in an embodiment, the main unit of ventilation device 100 further includes an expiratory valve 50, which includes a gas inlet interface 51 and a gas outlet interface 52, wherein the gas inlet interface 51 is connected with the expiratory branch and receive an exhaled gas of a patient through the expiratory branch, wherein the gas inlet interface 51 and the output interface 22 are located on a same side of the housing 10. In this embodiment, a connection pipeline of the gas inlet interface 51 of the expiratory valve 50 and a connection pipeline of the gas path assembly 20 are located on a same side of the housing 10, and are separated from a connection cable of the portable medical device 200, which can avoid cluttered connection pipelines and facilitate the use of the main unit of ventilation device 100.

In an embodiment, the gas outlet interface 51 is partially located on the back plate A2 of the housing, and the gas outlet interface 52 is partially located on the right plate A6 of the housing. In this embodiment, when a part of the gas outlet interface 51 is blocked by a wall or another medical device or a quilt, for example, when a part of the gas outlet interface 51, which part is located on the back plate A2, is blocked by a wall or another medical device or a quilt, a part of the gas outlet interface 52, which part is located on the right plate A6 can still ventilate, so as not to cause the patient to be unable to exhale.

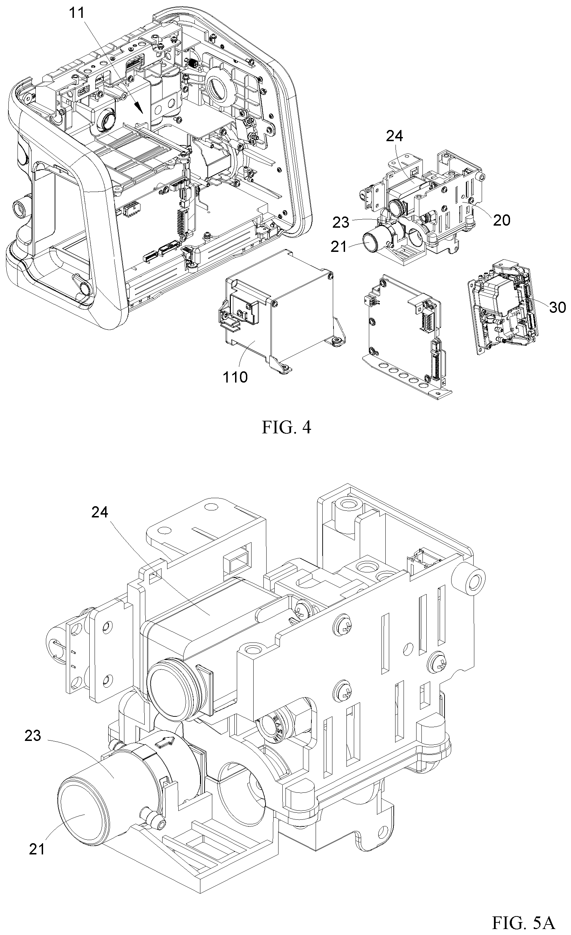

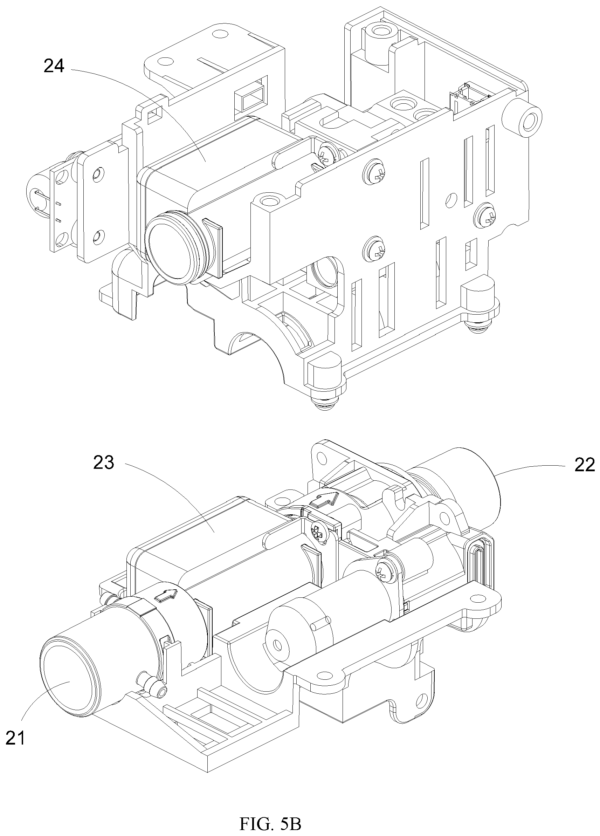

As shown in FIG. 4 , FIG. 5A and FIG. 5B, in an embodiment, the main unit of ventilation device 100 further includes a turbine mechanism 110, and the gas output assembly 23 is provided with an input interface 21 and an output interface 22. The oxygen control assembly 24 is connected with the turbine mechanism 110. The oxygen control assembly 24 is further connected with the oxygen supply apparatus, so as to converge oxygen, which is supplied by the oxygen supply apparatus, into the turbine mechanism 110. The input interface 21 is connected with the turbine mechanism 110. A gas, which is outputted by the turbine mechanism 110, enters into the gas output assembly 23 through the input interface 21.

It should be noted that the oxygen control assembly 24 being connected with the gas output assembly 23, can be that the oxygen control assembly 24 and the gas output assembly 23 are directly connected with each other; or the oxygen control assembly 24 and the gas output assembly 23 are in directly connected with each other. For example, in some embodiments, the main unit of ventilation device 100 further includes a turbine mechanism 110, wherein the oxygen control assembly 24 and the gas output assembly 23 are both connected with the turbine mechanism 110, and the oxygen control assembly 24 and the gas output assembly 23 are connected with each other through the turbine mechanism 110.

In an embodiment, the oxygen control assembly 24 includes a proportional valve and a first flow amount sensor, wherein the proportional valve is configured to adjust a flow amount of oxygen, and the first flow amount sensor is configured to monitor the flow amount of oxygen.

In an embodiment, the gas output assembly 23 includes a release valve and a second flow amount sensor, wherein when a pressure of an output gas is greater than a preset value, the release valve opens to release part of the gas to balance the pressure, and the second flow amount sensor is configured to monitor the flow amount of the output gas.

As shown in FIG. 2, in an embodiment, the main unit of ventilation device 100 further includes an oxygen interface 60, which is connected with the oxygen control assembly 24 through a pipeline. The oxygen interface 60 is connected with an oxygen supply apparatus to deliver oxygen which is supplied by the oxygen supply apparatus to the oxygen control assembly 24, wherein the oxygen interface 60 and the cable interface 202 are located on a same side of the housing 10. In this embodiment, by separately arranging the oxygen interface 60 and the oxygen control assembly 24, the oxygen interface 60 and the oxygen control assembly 24 can be flexibly arranged, and an internal space of the housing 10 can be reasonably and effectively utilized to achieve the miniaturization of the main unit of ventilation device 100.

In an embodiment, the accommodation cavity 12 has a cavity opening, the oxygen interface 60 and the cavity opening are located on a same side of the housing 110, and the accommodation cavity 12 is adjacent to the bottom plate A4 and the back plate A2.

As shown in FIGS. 2 and 3, in an embodiment, the housing 10 includes a first side plate, a second side plate and a third side plate, which are on different surfaces and are interconnected with one another, wherein the accommodation cavity 12 is formed by recessing inward of one of the first side plate, the second side plate and the third side plate.

Taking the example that the first side plate is the left plate A5, the second side plate is the back plate A2, and the third side plate is the top plate A3, the accommodation cavity 12 can be formed by recessing inward the left plate A5, or recessing inward the back plate A2, or recessing inward the top plate A3. Of course, this is just an exemplary explanation. According to an actual usage requirement, the accommodation cavity 12 can be formed by recessing inward any one of the front plate A1, the back plate A2, the top plate A3, the left plate A5 and the right plate A6, and the specific shape can be determined according to an actual design requirement.

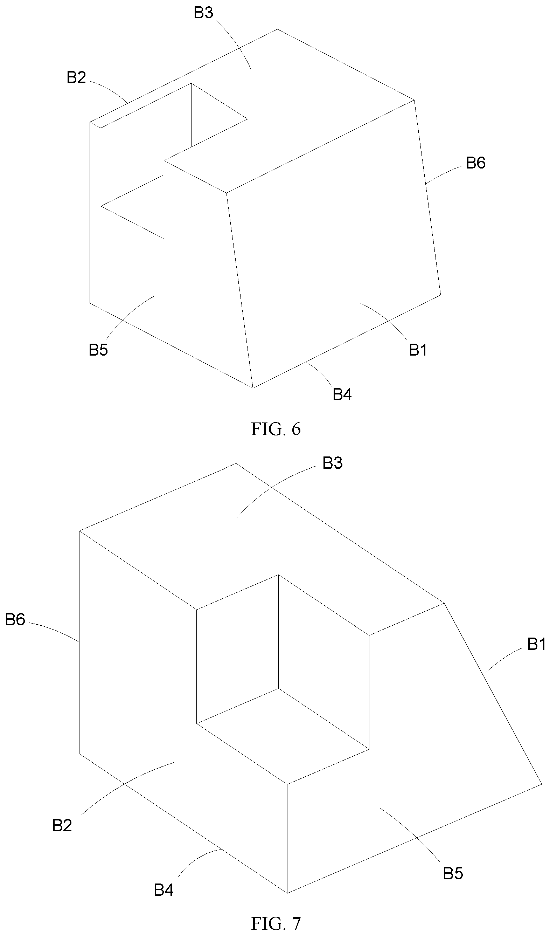

In an embodiment, the accommodation cavity 12 is formed recessing inward two of the first side plate, the second side plate and the third side plate. Taking that the first side plate is the left plate A5, the second side plate is the back plate A2, and the third side plate is the top plate A3 as shown in FIG. 6 for example, the accommodation cavity 12 can be formed by recessing inward the left plate A5 and the top plate A3, or by recessing inward the left plate A5 and the top plate A3, or by recessing inward the back plate A2 and the top plate A3. Of course, this is just an exemplary explanation. According to an actual usage requirement, the accommodation cavity 12 can be formed by recessing inward any adjacent two of the front plate A1, the back plate A2, the top plate A3, the left plate A5 and the right plate A6, and the specific shape can be determined according to an actual design requirement.

In an embodiment, the accommodation cavity 12 is formed by recessing inward three of the first side plate, the second side plate and the third side plate. Taking that the first side plate is the left plate A5, the second side plate is the back plate A2, and the third side plate is the top plate A3 as shown in FIG. 7 for example, the accommodation cavity 12 is formed by recessing inward the left plate A5, the back plate A2, and the top plate A3. Of course, this is just an exemplary explanation. According to an actual usage requirement, the accommodation cavity 12 can be formed by recessing inward any adjacent three of the front plate A1, the back plate A2, the top plate A3, the left plate A5 and the right plate A6, and the specific shape can be determined according to an actual design requirement.

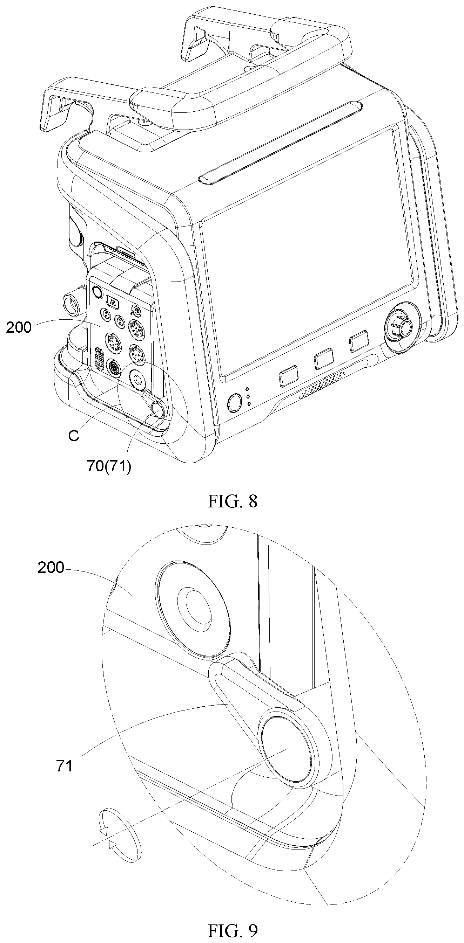

As shown in FIGS. 8 and 9, in an embodiment, the main unit of ventilation device 100 further includes a first position-limiting structure 70, which is connected with the housing 10. The first position-limiting structure 70 is configured to cooperate with the portable medical device 200, so as to enable the portable medical device 200 to be stably accommodated inside the accommodation cavity 12. In this embodiment, a cooperation between the first position-limiting structure 70 and the portable medical device 200 can prevent the portable medical device 200 from falling out of the accommodation cavity 12 during a transportation of the main unit of ventilation device 100.

In an embodiment, the first position-limiting structure 70 includes a position-limiting member 71, which is movably connected with the housing 10, and has an open position and a close position. When the position-limiting member 71 moves to the open position, the portable medical device 200 can be freely assembled into and disassembled out of the accommodation cavity 12. When the position-limiting member 71 moves to the close position, the position-limiting member 71 abuts against the portable medical device 200 to limit the portable medical device 200 from being disassembled out of the accommodation cavity 12.

As shown in FIG. 9, in an embodiment, the position-limiting member 71 is arranged at an edge of the accommodation cavity 12 and is rotatably connected with the housing 10. When the position-limiting member 71 is rotated to the open position, the position-limiting member 71 is located outside a contour of the accommodation cavity 12. When the position-limiting member 71 is rotated to the close position, the position-limiting member 71 is partially located within the contour of the accommodation cavity 12, so as to limit the portable medical device 200 from being disassembled out of the accommodation cavity 12. In this embodiment, when the portable medical device 200 needs to be accommodated into the accommodation cavity 12, the position-limiting member 71 is first rotated to the open position. At this time, the position-limiting member 71 will not block the accommodation cavity 12, and the portable medical device 200 can be accommodated into or disassembled out of the accommodation cavity 12 at will. After the portable medical device 200 is accommodated into the accommodation cavity 12, the position-limiting member 71 is rotated to the close position. At this time, the position-limiting member 71 blocks the portable medical device 200 to prevent the portable medical device 200 from falling off from the accommodation cavity 12. When the portable medical device 200 needs to be disassembled from the accommodation cavity 12, the position-limiting member 71 is first rotated to the open position, and then the portable medical device is disassembled from the accommodation cavity 12 .

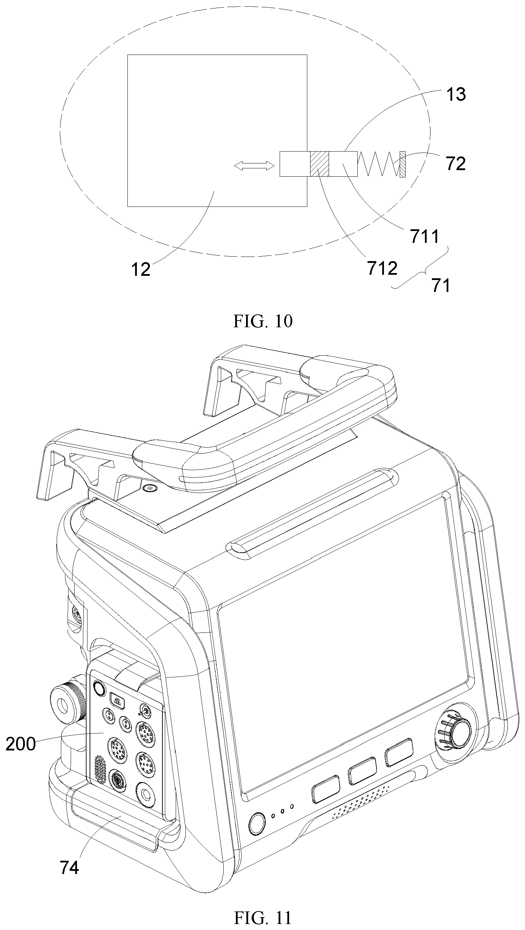

It should be noted that the position-limiting member 71 is not limited to being set in a manner of being rotatably connected with the housing 10. For example, in another embodiment, as shown in FIG. 10, an inner wall of the accommodation cavity 12 is provided with a slide groove, and the outer side surface of the housing 10 is provided with an operation port 13, which is connected with the slide groove. The first position-limiting structure 70 includes a position-limiting member 71 and a first elastic member 72. The position-limiting member 71 includes a position-limiting part 711 and an operation part 712 which is connected with the position-limiting part 711. The position-limiting part 711 penetrates into and is slidably arranged inside the slide groove, and the operation part 712 penetrates into and is arranged inside the operation port 13. The position-limiting part 711 can be driven to move to the open position by operating the operation part 712. In the open position, the position-limiting part 711 is located inside the slide groove. The first elastic member 72 is arranged inside the slide groove and abuts against the position-limiting part 711. The first elastic member 72 is configured to provide a drive force to drive the position-limiting part 711 to move to the close position. In the close position, the position-limiting part 711 partially extends into the accommodation cavity 12 to limit the portable medical device 200 from being disassembled out of the accommodation cavity 12. In this embodiment, when the portable medical device 200 needs to be accommodated into the accommodation cavity 12, the operation part 712 is first pushed to move the position-limiting member 71 to the open position. At this time, the position-limiting part 711 will not block the accommodation cavity 12, and the portable medical device 200 can be accommodated into or disassembled out of the accommodation cavity 12 at will. After the portable medical device 200 is accommodated into the accommodation cavity 12, the operation part 712 is released. Now the position-limiting part 711 extends into the accommodation cavity 12 under an elastic force of the first elastic member 72. At this time, the position-limiting part 711 blocks the portable medical device 200 to prevent the portable medical device 200 from falling off from the accommodation cavity 12. When the portable medical device 200 needs to be disassembled from the accommodation cavity 12, the operation part 712 is first pushed to be moved the position-limiting member 71 to the open position, and then the portable medical device can be disassembled from the accommodation cavity 12.

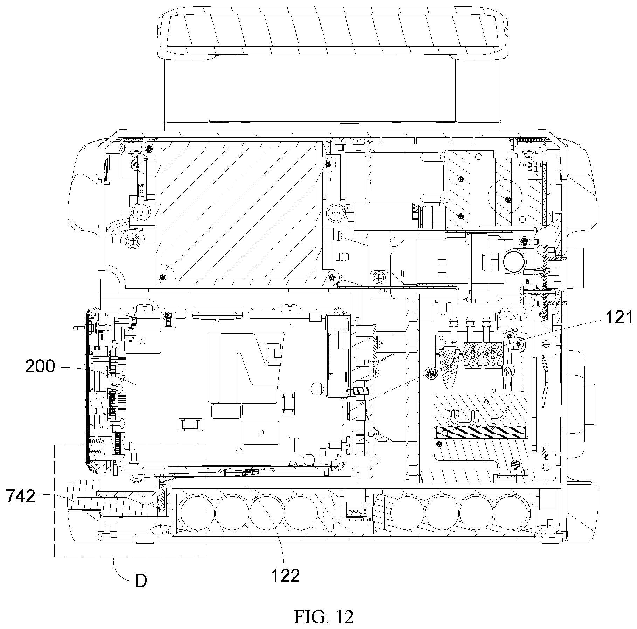

It should be noted that the first position-limiting structure 70 is not limited to the above arrangement method. For example, in another embodiment, as shown in FIGS. 11 to 15, the housing 10 includes a top part, a bottom part, and a side part which surrounds the top part and the bottom part. The accommodation cavity 12 is formed by recessing the side part towards an interior of the housing 10. The accommodation cavity 12 includes a bottom wall 121, and a first side wall 122 which is adjacent to the bottom wall 121. The bottom wall 121 is opposite to the cavity opening of the accommodation cavity 12. Preferably, the first side wall 122 is a side wall that supports a bottom of the portable medical device 200, and the portable medical device 200 abuts against the first side wall 122 under an action of gravity. The first position-limiting structure 70 includes a protrusion 73 and a lifting assembly 74. The protrusion 73 protrudes from the first side wall 122 and is snapped into a second position-limiting structure 203 which is arranged at the portable medical device 200, so as to limit the portable medical device 200 from being disassembled out of the accommodation cavity 12. The lifting assembly 74 is configured to lift the portable medical device 200, when subjected to an external force, so as to enable the second position-limiting structure 203 of the portable medical device 200 to be separated from the protrusion 73. In this embodiment, when the portable medical device 200 needs to be assembled, the portable medical device 200 only needs to be pushed into the accommodation cavity 12 until the protrusion 73 is snapped into the second position-limiting structure 203. At this time, under the limitation of the protrusion 73, the portable medical device 200 will not fall off from the accommodation cavity 12. When the portable medical device 200 needs to be disassembled from the accommodation cavity 12, the operator operates the lifting assembly 74 to lift the portable medical device 200, so as to enable the second position-limiting structure 203 of the portable medical device 200 to be separated from the protrusion 73. Then the position-limiting effect of the protrusion 73 is lost, and the portable medical device 200 can be directly disassembled out of the accommodation cavity 12.

In an embodiment, the first position-limiting structure 70 further includes an outer housing 75, wherein the outer housing 75 is provided with a protrusion 73, the lifting assembly 74 includes a lifting member 741 and a pushing member 742, wherein the lifting member 741 is slidably connected with the outer housing 75 along a first direction X, and the lifting member 741 is configured to lift the portable medical device 200 along the first direction X. The pushing member 742 is slidably connected with the outer housing 75 along a second direction Y. The pushing member 742 is configured to drive the lifting member 741 to ascend, so as to lift the portable medical device 200. An angle exists between the second direction Y and the first direction X. When the portable medical device 200 needs to be disassembled from the accommodation cavity 12, the operator manually pushes the pushing member 742 along the second direction Y, and then the pushing member 742 drives the lifting member 741 to lift the portable medical device 200, so as to separate the portable medical device 200 from the protrusion 73.

In an embodiment, the lifting assembly 74 further includes a first restoration member, which is arranged at the outer housing 75 and configured to provide a drive force to drive the lifting member 741 to descend. In this embodiment, when the operator removes the external force applied to the pushing member 742, the lifting member 741 descends under an action of the first restoration member, so that the portable medical device 200 can be again assembled into the accommodation cavity 12 and then snap into the protrusion 73. The first restoration member may be, but is not limited to, a spring.

It should be noted that, in another embodiment, the lifting assembly 74 may not be provided with the first restoration member. When the portable medical device 200 is accommodated into the accommodation cavity 12, the portable medical device 200 may also drive the lifting member 741 to descend by relying on its gravity.

In an embodiment, the lifting assembly 74 further includes a second restoration member, which is arranged at the outer housing 75 and configured to provide a drive force to drive the pushing member 742 to restore. In this embodiment, when an external force applied by an operator on the pushing member 742 is removed, the pushing member 742 moves to an initial position under an action of the second restoration member, making it convenient for a subsequent operator to push the pushing member 742 to lift the lifting member 74. The second return member may be, but is not limited to, a spring.

It should be noted that, in another embodiment, the lifting assembly 74 may not be provided with a second restoration member. In this embodiment, the lifting member 741 is provided with a first inclined surface 7411, and the pushing member 742 is provided with a second inclined surface 7421. When the lifting member 741 descends, the lifting member 741 can push the pushing member 742 to restore through a cooperation between the first inclined surface 7411 and the second inclined surface 7421.

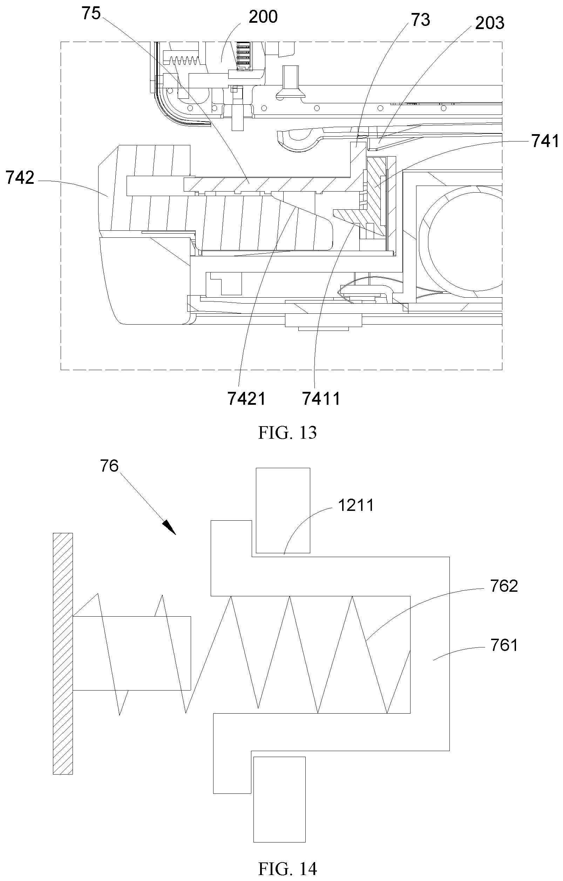

As shown in FIG. 14, in an embodiment, the first position-limiting structure 70 further includes a pushing-out mechanism 76, which is arranged at the bottom wall 121, and is configured to provide a drive force to push the portable medical device 200 out of the accommodation cavity 12. It should be noted that, in this embodiment, the pushing-out mechanism 76 only pushes a portion of the portable medical device 200 out from the accommodation cavity 12, making it convenient for an operator to grasp the pushed-out portion of the portable medical device 200 for taking out the entire portable medical device 200. Of course, in some other embodiments, the pushing-out mechanism 76 may also push the entire portable medical device 200 out of the accommodation cavity 12, and the specific method may be determined according to an actual design requirement.

In an embodiment, the bottom wall 121 is provided with a hole 1211, and the pushing-out mechanism 76 includes a pushing-out member 761 and a second elastic member 762. The pushing-out member 761 is arranged inside the hole 1211 and a portion of the pushing-out member 761 can extend along the hole 1211. The second elastic member 762 is arranged inside the inner cavity 11 and abuts against the pushing-out member 761. The second elastic member 762 is configured to provide a drive force for the pushing-out member 761 to extend out of the hole 1211. The second elastic member 762 may be, but is not limited to, a spring. While the portable medical device 200 is accommodated into the accommodation cavity 12, it abuts against the pushing-out member 761 and gradually compresses the second elastic member 762. When the portable medical device 200 is lifted by the lifting member 741 and separated from the protrusion, the compressed second elastic member 762 pushes the portable medical device 200 out through an elastic force. In this embodiment, the pushing-out mechanism 76 has a simple structure, which is conducive to simplifying mechanism, reducing mechanism complexity, and improving mechanism reliability.

It should be noted that the pushing-out mechanism 76 is not limited to the above-mentioned pushing-out member 761 in combination with the second elastic member 762. For example, in some other embodiments, the pushing-out mechanism 76 may include a motor, a transmission mechanism and a pushing-out member 761. The pushing-out member 761 is connected with the motor through the transmission mechanism. When the motor is running, the transmission mechanism drives the pushing-out member 761 to push the portable medical device 200 out of the accommodation cavity 12. The transmission mechanism may be, but is not limited to, a crank-connection rod mechanism, a gear rack pair, or a cam transmission mechanism. In some other embodiments, the pushing-out mechanism 76 may also adopt a cylinder push rod, an electric push rod, an electromagnet mechanism, etc.

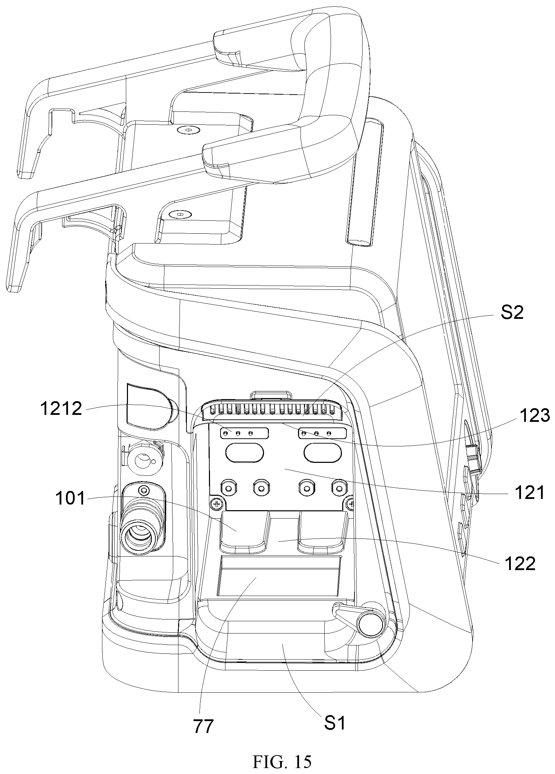

It should be noted that the first position-limiting structure 70 is not limited to the above arrangement method. For example, in another embodiment, as shown in FIGS. 15 to 18, the accommodation cavity 12 includes a bottom wall 121, and a first side wall 122 and a second side wall 123 which are arranged on opposite sides of the bottom wall 121, wherein the bottom wall 121 is opposite to the cavity opening of the accommodation cavity 12. The first position-limiting structure 70 includes a blocking part 77 which is arranged at the first side wall 122, and the second position-limiting structure 203 includes an elastic sheet 2031 and an abutting part 2032. The elastic sheet 2031 includes a first end, and a second end which is opposite to the first end. The first end of the elastic sheet 2031 is connected with the portable medical device 200, and the abutting part 2032 is connected with the elastic sheet 2031. When the portable medical device 200 is accommodated inside the accommodation cavity 12, the abutting part 2032 abuts against the blocking part 77, so as to limit the portable medical device 200 from being disassembled from. The abutting part 2032 can be separated from the blocking part 77 by pressing the elastic sheet 2031.

Optionally, the blocking part 77 is a groove arranged at the first side wall 122, and the abutting part 2032 is inserted into the groove. Of course, the blocking part 77 is not limited to being a groove, but may also be a protrusion, and the abutting part 2032 abuts against said protrusion.

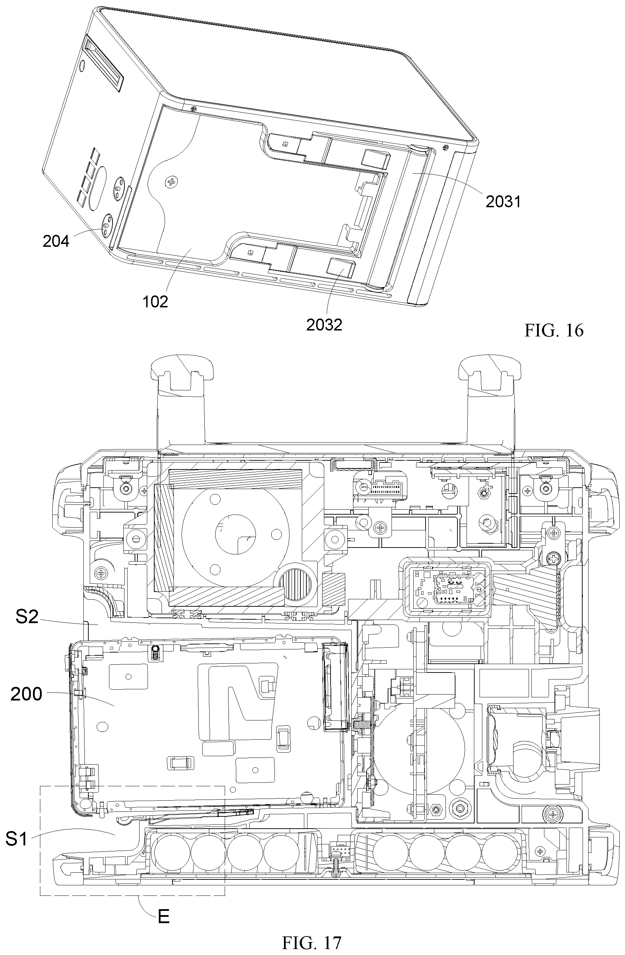

As shown in FIG. 15 and FIG. 17, in an embodiment, the first side wall 122 is provided with a first hand-holding position S1 at an edge of the accommodation cavity 12. In an embodiment, the second end of the elastic sheet 2031 is exposed at the first hand-holding position S1. The first side wall 122 obliquely extends outward at an opening of the accommodation cavity 12, so as to form the first hand-holding position S1. In this embodiment, by setting the first hand-holding position S1, it is convenient for an operator to disassemble and assemble the portable medical device 200.

As shown in FIG. 15 and FIG. 17, in an embodiment, the second side wall 123 is provided with a second hand-holding position S2 at an edge of the accommodation cavity 12. Similarly, by setting the second hand-holding position S2, a cooperation between the second hand-holding position S2 and the first hand-holding position S1 facilitates the operator to disassemble and assemble the portable medical device 200. Specifically, when the portable medical device 200 needs to be disassembled from the accommodation cavity 12, the operator inserts the thumb into the first hand-holding position S1, and inserts the index finger and the middle finger into the second hand-holding position S2. This is just an example and not a specific limitation. The finger inserted into the first hand-holding position S1 may be at least one of the four fingers other than the thumb. After the index finger and the middle finger are inserted into the second hand-holding position S2, the index finger and the middle finger press the second end of the elastic sheet 2031 until the abutting part 2032 is disengaged from the blocking part 77, and then the thumb, the index finger and the middle finger apply a force to pull the portable medical device 200 out of the accommodation cavity 12.

As shown in FIGS. 15 and 16, in an embodiment, the accommodation cavity 12 includes a bottom wall 121, which is opposite to the cavity opening of the accommodation cavity 12. The bottom wall 121 is provided with a first interface assembly 1212, and the first interface assembly 1212 is electrically connected with a second interface assembly 204, which is arranged at the portable medical device 200. In this embodiment, the portable medical device 200 can be powered by a battery arranged inside the main unit of ventilation device 100 and/or communicated with a control assembly which is arranged inside the main unit of ventilation device 100 through a cooperation of the first interface assembly 1212 and the second interface assembly 204.

As shown in FIGS. 15 and 16, in an embodiment, the housing 10 includes a top part and a bottom part, and a side part which surrounds the top part and the bottom part, wherein the accommodation cavity 12 is formed by recessing the side part into an interior of the housing 10, and the accommodation cavity 12 includes a bottom wall 121 and a side wall which surrounds the bottom wall 121, and the bottom wall 121 is opposite to the cavity opening of the accommodation cavity 12. The side wall is provided with a first guide structure 101, which extends along an assembling direction of the portable medical device 200. The first guide structure 101 is configured to cooperate with a second guide structure 102, which is arranged at the portable medical device 200, so as to form a guide when the portable medical device 200 is accommodated into the accommodation cavity 12. In this embodiment, the portable medical device 200 can be assembled at a correct position through the guide of the first guide structure 101 and the second guide structure 102. In addition, the cooperation between the first guide structure 101 and the second guide structure 102 can also limit the portable medical device 200 from shaking in a direction perpendicular to the assembling direction.

Optionally, the first guide structure 101 is a protrusion 73, and the second guide structure 102 is a groove. It should be noted that positions of the protrusion 73 and the groove can be swapped, that is, the first guide structure 101 is a groove, and the second guide structure 102 is a protrusion 73.

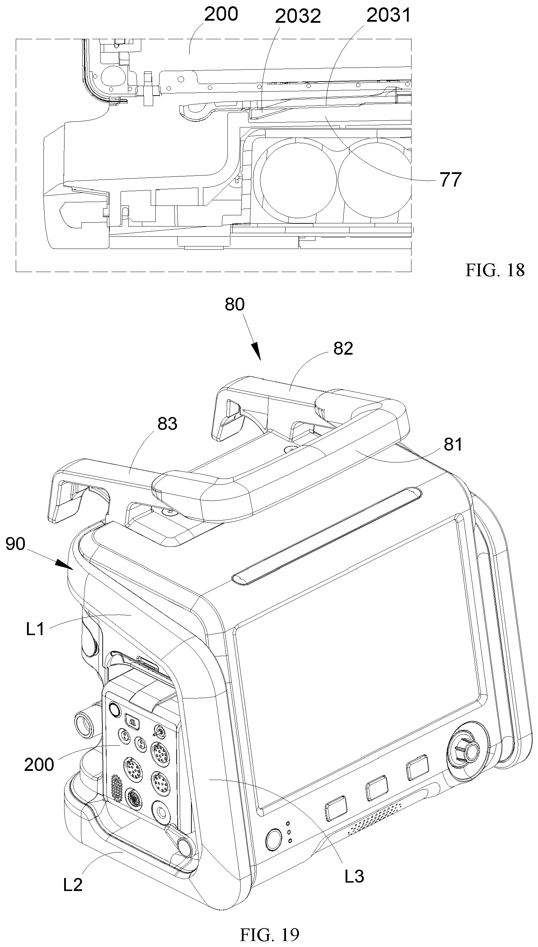

As shown in FIG. 19, in an embodiment, the main unit of ventilation device 100 further includes a handle 80, which is connected with the housing 10, and an indication structure is arranged at the handle 80. In this embodiment, by providing an indication structure, it is convenient for the user to identify the handle 80 and/or the main unit of ventilation device 100.

In an embodiment, the indication structure includes a light source, and the light source indicates the user by emitting light. Of course, the indication structure is not limited to the above-mentioned arrangement method. In some other embodiments, the indication structure includes a silk-screen ink layer with reflective powder added, and the silk-screen ink layer sends an indication to the user through reflection. In some other embodiments, the indication structure includes a reflective strip, which is provided with a reflective structure, wherein the reflective strip indicates the user by reflecting light.

In an embodiment, the main unit of ventilation device 100 includes a front side which is provided with an operation panel, the handle 80 includes a front side surface which faces the front side of the main unit of ventilation device 100, and the indication structure is arranged on the front side surface of the handle 80. Of course, the indication structure is not limited to being arranged on the front side surface of the handle 80, and can also be arranged on other side surfaces of the handle 80, depending on an actual design requirement.

As shown in FIG. 19, in an embodiment, the handle 80 includes a cross rod 81, a first connection rod 82, and a second connection rod 83. The cross rod 81 is located at a top part of the housing 10 and extends along a left-right direction of the housing 10. The indication structure is arranged at the cross rod 81, and the cross rod 81 includes a first end and a second end. The first connection rod 82 is connected with a first end of the cross rod 81 and the housing 10, and the second connection rod 83 is connected with a second end of the cross rod 81 and the housing 10. The cross rod 81, the first connection rod 82, and the second connection rod 83 together form a U-shaped structure.

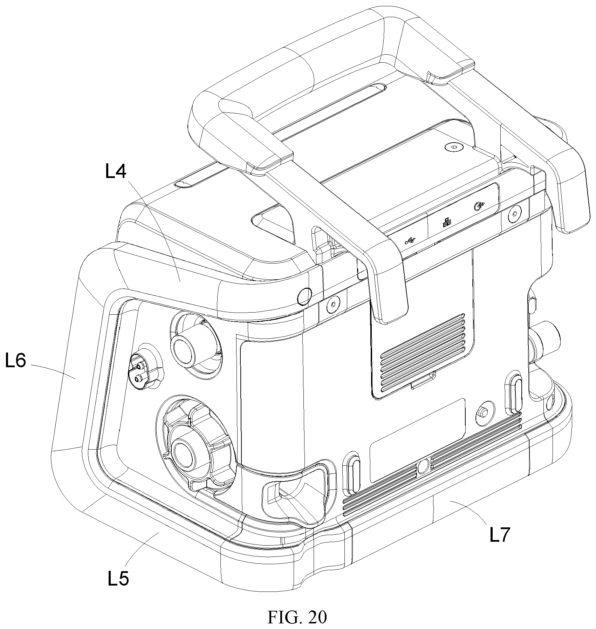

As shown in FIGS. 19 and 20, in an embodiment, the main unit of ventilation device 100 further includes a protective member 90, which is connected with the housing 10, and configured to protrude from a surface of the housing 10 at a height greater than a height at which the portable medical device 200 protrudes from said surface of the housing 10. In this embodiment, when the main unit of ventilation device 100 is hit or accidentally dropped during transportation, the protective member 90 is hit first, thereby protecting the portable medical device 200 and preventing the portable medical device 200 from being damaged by impact. The protective member 90 is made of elastic material. Optionally, the protective member 90 may be, but is not limited to, made of silicone material.

In an embodiment, the housing 10 includes a front side, a back side, a left side and a right side, wherein the left side includes a first top edge L1, a first bottom edge L2, and a first side edge L3 which is adjacent to the front side, the right side includes a second top edge L4, a second bottom edge L5, and a second side edge L6 which is adjacent to the front side, wherein the back side includes a third bottom edge L7. The protective member 90 is in a shape of an elongated strip, and extends sequentially along the first top edge L1, the first side edge L3, the first bottom edge L2, the third bottom edge L7, the second bottom edge L5, the second side edge L6, and the second top edge L4.

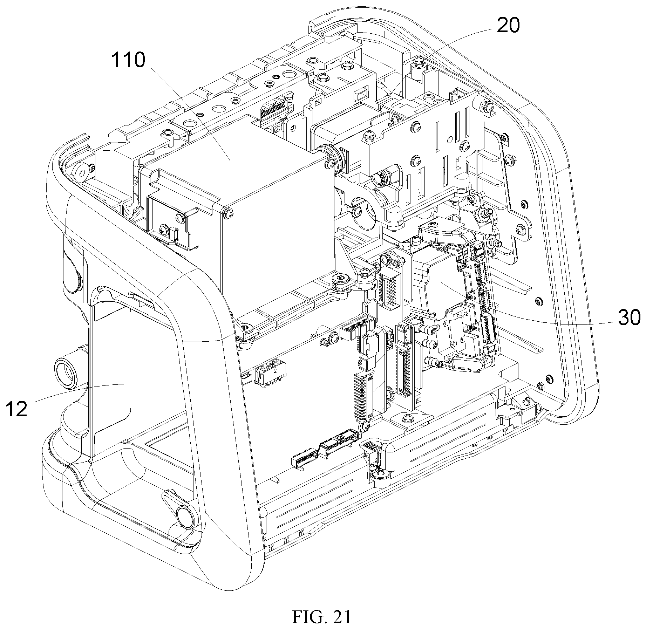

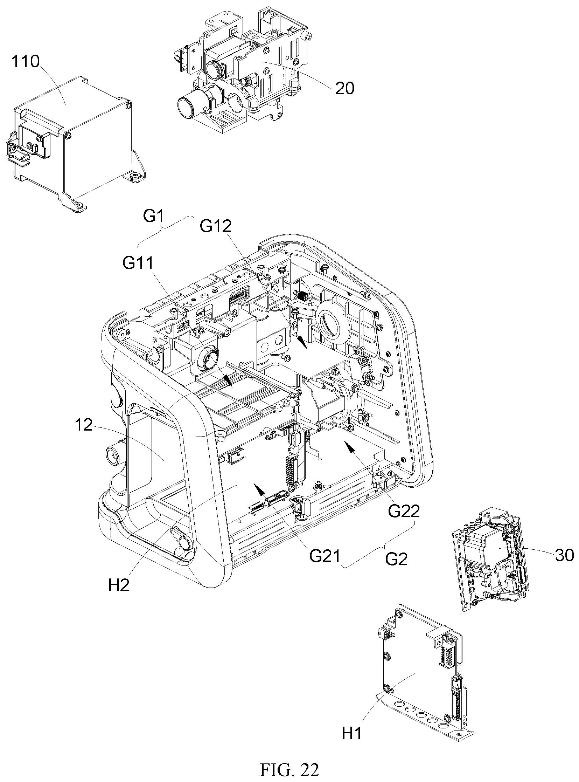

As shown in FIGS. 21 and 22, in an embodiment, the main unit of ventilation device 100 further includes a turbine mechanism 110, which is arranged inside the inner cavity 11 and is connected with the gas path assembly 20. The turbine mechanism 110 and the accommodation cavity 12 are arranged side by side in a height direction of the main unit of ventilation device 100, and the gas path assembly 20 and the accommodation cavity 12 are arranged diagonally, that is, the gas path assembly 20 and the expiratory valve 50 are arranged side by side in the height direction. In this embodiment, the turbine mechanism 110 and the gas path assembly 20 are reasonably arranged, such that an internal space of the housing 10 can be effectively and reasonably utilized, such that a size of the main unit of ventilation device 100 can be reduced, which enables the main unit of ventilation device 100 to satisfy a miniaturization requirement.

It should be noted that it is not limited to that the turbine mechanism 110 and the accommodation cavity 12 are arranged side by side in a height direction of the main unit of ventilation device 100, the pressure monitoring assembly 30 and the accommodation cavity 12 can also be arranged side by side in a width direction of the main unit of ventilation device 100. As long as one of the turbine mechanism 110, the gas path assembly 20 and the pressure monitoring assembly 30 is arranged side by side with the accommodation cavity 12 in the height direction of the main unit of ventilation device 100, and another of the turbine mechanism 110, the gas path assembly 20 and the pressure monitoring assembly 30 is arranged side by side with the accommodation cavity 12 in the width direction of the main unit of ventilation device 100, and the remaining one of the turbine mechanism 110, the gas path assembly 20 and the pressure monitoring assembly 30 is arranged diagonally with the accommodation cavity 12, the size of the main unit of ventilation device 100 can be reduced, which enables the main unit of ventilation device 100 to satisfy a miniaturization requirement.

In an embodiment, the main unit of ventilation device 100 includes an upper region G1 and a lower region G2, wherein the upper region G1 includes a first region G11, and a second region G12 which is arranged side by side with the first region G11, the lower region G2 includes a third region G21, and a fourth region G22 which is arranged side by side with the third region G21, wherein the first region G11 and the third region G21 are arranged side by side in a height direction of the main unit of ventilation device 100, while the second region G12 and the fourth region G22 are also arranged side by side in a height direction of the main unit of ventilation device 100. The main unit of ventilation device 100 further includes an expiratory valve 50. The housing 10 is recessed inward to form an assembly cavity, and the expiratory valve 50 can be assembled inside the assembly cavity, which assembly is capable of being disassembled; wherein, the turbine mechanism 110 is located inside the first region G11, the gas path assembly 20 is located inside the second region G12, the accommodation cavity 12 is located inside the third region G21, and the assembly cavity is located inside the fourth region G22.

In an embodiment, the oxygen control assembly 24 is connected with the turbine mechanism 110, and is configured to input oxygen into the turbine mechanism 110, so as to mix said oxygen with a gas inside the turbine mechanism 110 before entering the gas output assembly 23. It should be noted that, in another embodiment, the main unit of ventilation device 100 is not provided with the turbine mechanism 110, the oxygen control assembly 24 is connected with the gas output assembly 23, and configured to input oxygen into the gas output assembly 23.

In an embodiment, the main unit of ventilation device 100 further includes a first control board H1 and a second control board H2, which are located inside the housing 10, wherein the first control board H1 is located between the accommodation cavity and the front plate A1 of the housing 10, and the second control board H2 is located below the gas output assembly 23. The first control board H1 is configured to, but not limited to, control the portable medical device 200. The second control board H2 is used to, but not limited to, control the turbine mechanism 110.

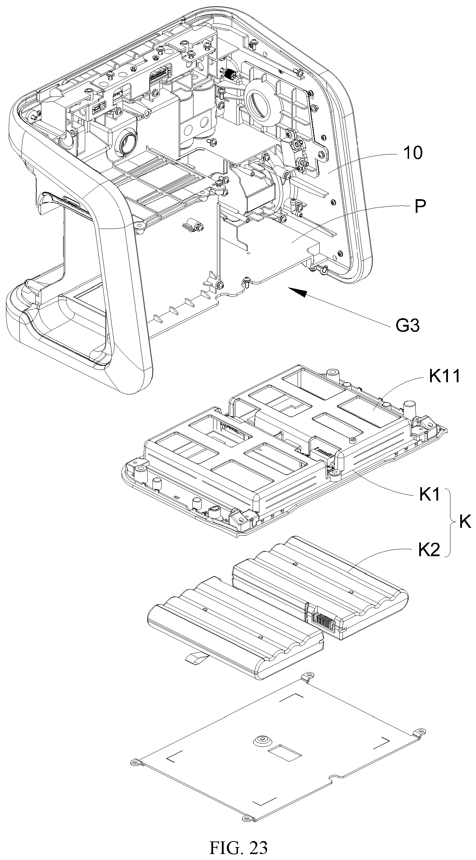

As shown in FIG. 23, in an embodiment, the main unit of ventilation device 100 further includes a support plate P and a battery assembly K. The support plate P is horizontally arranged inside the housing 10. The upper region G1 and the lower area G2 mentioned above are above the support plate P. The bottom region G3 is formed below the support plate P. The battery assembly K is accommodated inside the bottom region G3, and configured to supply power required for an operation of the main unit of ventilation device 100.

In this embodiment, the battery assembly K is separated from the gas path assembly 20, the pressure monitoring assembly 30 and the turbine mechanism 110 mentioned above by providing a support plate P, which is beneficial to a protection between these two groups of assemblies, reduces a mutual influence between two groups of assemblies, and improves safety performance. Secondly, by arranging the bottom region G3 to accommodate the battery assembly K, when the battery assembly K is accommodated inside the bottom region G3, a gravity center of the main unit of ventilation device 100 as a whole can be lowered, so that the main unit of ventilation device 100 can be stably placed on a support surface.

In an embodiment, the bottom region G3 and the battery assembly K are both flat, and the battery assembly K is laid flat on the bottom region G3. This implementation is helpful to lower the gravity center of the main unit of ventilation device 100, so that the main unit of ventilation device 100 can be placed more stably, and the height of the entire main unit of ventilation device 100 can be reduced to satisfy a miniaturization requirement.

In an embodiment, the battery assembly K includes a battery protection shield K1 and a battery K2. The battery protection shield K1 is connected with the housing, and the battery K2 is accommodated inside the battery protection shield K1. Optionally, the battery protection shield K1 is provided with a hollow structure K11 for heat dissipation.

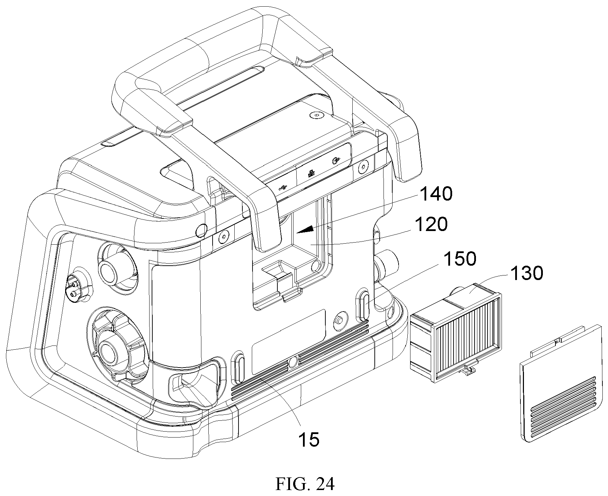

As shown in FIG. 24, in an embodiment, an air inlet 120 is arranged at a back part of the main unit of ventilation device 100, and the air inlet 120 is connected with the turbine mechanism 110, and air enters into the turbine mechanism 110 through the air inlet 120. It should be noted that the air inlet 120 is not limited to being arranged on the back of the main unit of ventilation device 100, and can actually be arranged according to a position of an inlet of the turbine mechanism 110. For example, in another embodiment, the gas inlet of the turbine mechanism 110 is located on the left side of the main unit of ventilation device 100. Correspondingly, the air inlet 120 can be arranged on the left plate A5 of the main unit of ventilation device 100.

As shown in FIG. 24, in an embodiment, the main unit of ventilation device 100 further includes a filter assembly 130, and an accommodation cavity 140 for the filter assembly is arranged at the back part of the main unit of ventilation device 100, and the cavity opening of the accommodation cavity 140 for the filter assembly is the air inlet 120, and the filter assembly 130 can be removably accommodated inside the accommodation cavity 140 for the filter assembly.

As shown in FIG. 24, in an embodiment, the main unit of ventilation device 100 further includes a bump 150, which protrudes from the back part of the housing 10. In this embodiment, when the main unit of ventilation device 100 is placed with its back part against a wall or another medical device, the back plate A2 of the main unit of ventilation device 100 can be separated by the bump 150 from the wall or another medical device, so as to prevent the air inlet 120 from being blocked by the wall or another medical device, thereby allowing the turbine mechanism 110 to have smooth air intake.

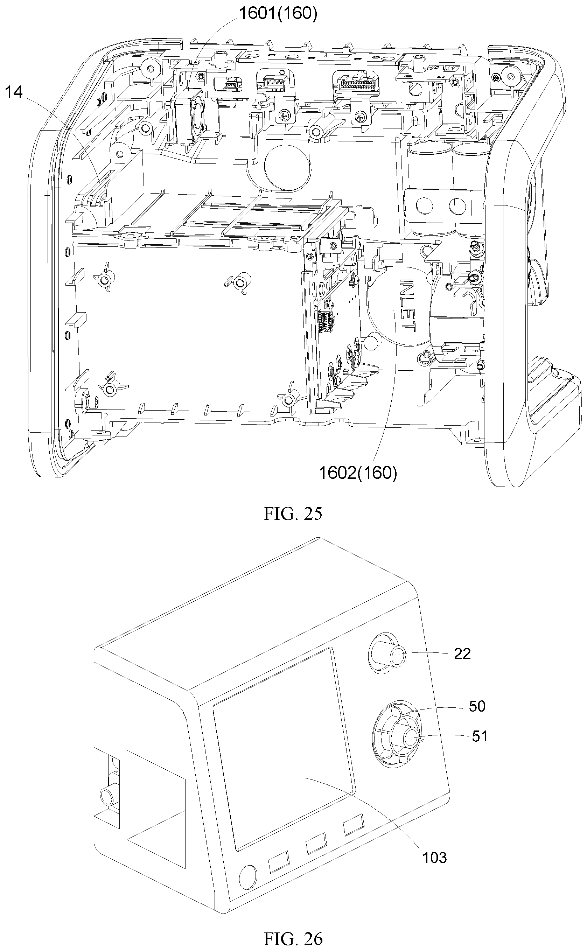

As shown in FIGS. 24 and 25, in an embodiment, the housing 10 includes a left side, a right side and a back part, wherein the housing 10 is provided with a gas inlet 14 and a gas outlet 15. The gas inlet 14 is located on the left side of the housing 10, and the gas outlet 15 is located on the back part of the housing 10. The main unit of ventilation device 100 further includes a gas drive assembly 160, which is arranged inside the inner cavity 11, and configured to drive a gas to enter into the inner cavity 11 from the gas inlet 14 and then exit out of the inner cavity 11 from the gas outlet 15.

In an embodiment, the gas inlet 14 is located at the first hand-holding position S1.

In an embodiment, the gas drive assembly 160 includes a first fan 1601 and a second fan 1602. The first fan 1601 is arranged on the back plate A2 of the housing 10, and the second fan 1602 is arranged at the gas outlet 15.

In an embodiment, the housing 10 includes a back plate A2, and a front plate A1 which is opposite to the back plate A2, wherein the back plate A2 of the housing 10, the turbine mechanism 110, the gas path assembly 20, and the pressure monitoring assembly 30, together form a first gas flow channel. The front plate A1 of the housing 10, the turbine mechanism 110, the gas path assembly 20, and the pressure monitoring assembly 30, together form a second gas flow channel. That is, after a gas enters into an interior of the housing 10 from the gas inlet 14, said gas flows along two channels. One gas flow enters into a gap between the back plate A2 and the turbine mechanism 110 under blocking and guidance of the turbine mechanism 110, and then flows through the gas path assembly 20 and the pressure monitoring assembly 30 sequentially before exiting out from the gas outlet 15. The other gas flow enters into a gap between the front plate A1 and the turbine mechanism 110 under blocking and guidance of the turbine mechanism 110, and then flows through the gas path assembly 20 and the pressure monitoring assembly 30 sequentially before exiting out from the gas outlet 15. In this embodiment, after the gas enters the interior of the housing 10 from the gas inlet 14, it can fully carry away heat inside the housing 10, thereby achieving a better heat dissipation effect.

In an embodiment, the main unit of ventilation device 100 further includes at least one portable medical device 200, which is removably accommodated inside the accommodation cavity 12. The portable medical device 200 may be, for example, a monitoring device without a display screen but with a power supply, a monitoring device without any display screen or power supply, a transport monitoring device with a display screen and a power supply, and the likes.

As shown in FIG. 2 , in an embodiment, the main unit of ventilation device 100 further includes a display screen 103, which is assembled at the front plate A1, and the display screen 103 is at least configured to display waveforms and monitoring parameters of respiration.

As shown in FIGS. 1 to 26, an embodiment of this disclosure further proposes a main unit of ventilation device. The proposed main unit of ventilation device 100 includes a housing 10, a display screen 103, a gas path assembly 20 and a pressure monitoring assembly 30. The housing 10 is provided with an inner cavity 11, wherein an outer side of the housing 10 is recessed inward to form an accommodation cavity 12. The display screen 103 is arranged at the housing 10. The gas path assembly 20 includes an oxygen control assembly 24 and an output interface 22. The oxygen control assembly 24 is connected with an oxygen supply apparatus, and the output interface 22 is connected with an inspiratory branch, and configured to deliver a gas to a patient through the inspiratory branch. The pressure monitoring assembly 30 is arranged inside the inner cavity 11, and at least configured to connect with the gas path assembly 20, so as to monitor a relevant parameter of an input gas and/or an output gas of the gas path assembly 20. Wherein the accommodation cavity 12 is configured to removably accommodate inside at least one portable medical device 200, wherein the portable medical device 200 includes an interface side 201, wherein the interface side 201 of the portable medical device 200 is provided with a cable interface 202. When the portable medical device 200 is accommodated inside the accommodation cavity 12, the output interface 22 and the cable interface 202 are located on two adjacent sides of the housing 10, wherein the output interface 22 is located on a side of the housing 10, on which side the display screen 103 is provided (as shown in FIG. 26).

The main unit of ventilation device 100 proposed in this embodiment, firstly, by arranging an accommodation cavity 12 on an outer side of the housing 10, the user can assemble a portable medical device 200 in the accommodation cavity 12 according to an actual use requirement, so as to expand a function of the ventilation device and broaden a scope of use. In some emergency situations, it can reduce the trouble of wiring and carrying instrument. Secondly, by arranging the portable medical device 200 to be removably accommodated inside the accommodation cavity 12, when the user does not need to use the extended function of the portable medical device 200, the portable medical device 200 can be removed and the main unit of ventilation device 100 can be used only as a ventilation device, thereby improving a flexibility of use and reducing an overall weight of the main unit of ventilation device 100. In addition, by arranging the portable medical device 200 to be accommodated inside the accommodation cavity 12, while enabling a cable interface 202 and an output interface 22 to be located on opposite sides of the housing 10, and locating the output interface 22 on a side of the housing 10, on which side the display screen 103 is provided; a connection pipeline of the gas path assembly 20, and a connection cable of the portable medical device 200 are separated, which can avoid cluttered connection pipeline and facilitate the use of the main unit of ventilation device 100.

In an embodiment, the gas path assembly 20 includes a gas output assembly 23, which is configured to receive a gas which is formed by mixing oxygen and air. The gas output assembly 23 includes the output interface 22.

In an embodiment, the main unit of ventilation device 100 further includes an expiratory valve 50, which includes a gas inlet interface 51, which is connected with an expiratory branch, wherein the gas inlet interface 51 and the output interface 22 are located on a same side of the housing 10. In this embodiment, the output interface 22 and the gas inlet interface 51 are arranged on a same side plate, so that the operator can conveniently plug in the inspiratory branch and the expiratory branch. In an embodiment, the operator can fix a plug-in end of the inspiratory branch and a plug-in end of the expiratory branch together, and arrange the ends to be spaced in consistent with that of the output interface 22 and the gas inlet interface 51. In this way, the operator can plug the inspiratory branch and the expiratory branch into place with the output interface 22 and the gas inlet interface 51 at one time, thereby improving operating efficiency.

The structures, connection relationships and beneficial effects of other components of the main unit of ventilation device 100 proposed in this embodiment can refer to the above embodiments and will not be described in detail here.

As shown in FIGS. 1 to 26, an embodiment of this disclosure further proposes a main unit of ventilation device 100. The proposed main unit of ventilation device 100 includes a housing 10, a gas path assembly 20 and a pressure monitoring assembly 30. The housing 10 is provided with an inner cavity 11. An outer side of the housing 10 is recessed inward to form an accommodation cavity 12. The gas path assembly 20 is arranged inside the inner cavity 11. The gas path assembly 20 includes an oxygen control assembly 24 and a gas output assembly 23. The oxygen control assembly 24 is connected with an oxygen supply apparatus. The gas output assembly 23 is configured to receive a gas which is formed by mixing oxygen and air. The gas output assembly 23 includes an output interface 22, which is connected with an inspiratory branch, and configured to deliver the gas to the patient through the inspiratory branch. The pressure monitoring assembly 30 is arranged inside the inner cavity 11, and at least configured to connect with the gas path assembly 20, so as to monitor a relevant parameter of an input gas and/or an output gas of the gas path assembly 20. The accommodation cavity 12 is configured to removably accommodate inside at least one portable medical device 200, and further includes an opening for inserting the portable medical device 200. The output interface 22 and at least a part of the opening are located on different sides of the housing 10. Exemplarily, the output interface 22 is located on the right plate of the housing 10, and at least a part of the opening is located on the left plate, the back plate, or the top plate of the housing 10.

In an embodiment, the portable medical device 200 is provided with a cable interface 202, and a second interface assembly 204 which is arranged opposite to the cable interface 202. The accommodation cavity 12 includes a cavity opening, and a bottom wall 121 which is opposite to the cavity opening. The bottom wall 121 is provided with a first interface assembly 1212, which is in communicative connection with the second interface assembly 204.

The main unit of ventilation device 100 proposed in this embodiment, firstly, by arranging an accommodation cavity 12 on an outer side of the housing 10, the user can assemble a portable medical device 200 in the accommodation cavity 12 according to an actual use requirement so as to expand the function of the ventilation device and broaden a scope of use. In some emergency situations, it can reduce the trouble of wiring and instrument carrying. Secondly, by arranging the portable medical device 200 to be removably accommodated inside the accommodation cavity 12, when the user does not need to use the extended function of the portable medical device 200, the portable medical device 200 can be removed and the main unit of ventilation device 100 can be used only as a ventilation device, thereby improving a flexibility of use and reducing an overall weight of the main unit of ventilation device 100.

The structures, connection relationships and beneficial effects of other components of the main unit of ventilation device 100 proposed in this embodiment can refer to the above embodiments and will not be described in detail here.

An embodiment of this disclosure further provides a ventilation device, which includes an inspiratory branch and the above-mentioned main unit of ventilation device 100, wherein the inspiratory branch is connected with the gas path assembly 20.

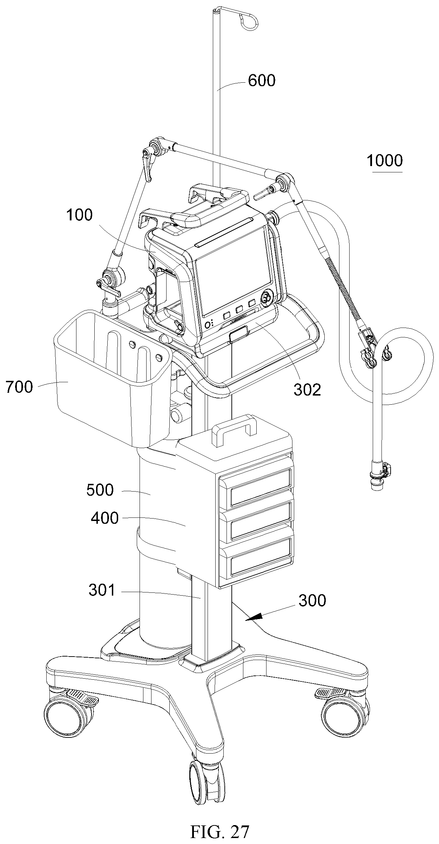

As shown in FIG. 27, an embodiment of this disclosure further proposes a medical device 1000, which includes a trolley 300, an infusion pump assembly 400 and the above-mentioned main unit of ventilation device 100. The trolley 300 includes a column 301, and a support frame 302 which is assembled at the column 301. The infusion pump assembly 400 is assembled at the column 301, the main unit of ventilation device 100 is placed on the support frame 302. In this embodiment, by arranged the infusion pump assembly 400 on the column 301, a space of the trolley 300 can be reasonably utilized, so that the medical device 1000 has a compact overall structure and occupies a reduced space. In addition, compared with the existing method of placing the infusion pump assembly 400 on the support frame 302, the infusion pump assembly 400 in this embodiment is assembled at the column 301, which can lower an overall gravity center of the medical device 1000, making the medical device 1000 more stable when moving. Moreover, the infusion pump assembly 400 is assembled at the column 301, which can reduce an impact of the infusion pump assembly 400 on the operator when the operator operates the main unit of ventilation device 100.

In an embodiment, the medical device 1000 further includes an oxygen cylinder 500, which is assembled at the column 302 and opposite to the infusion pump assembly 400. With this implementation, space can be reasonably utilized, so that the medical device 1000 has a compact structure and occupies a reduced space.

In an embodiment, the medical device 1000 further includes an infusion pole 600, which is assembled at the trolley 300 and is used to hang an infusion bag.

In an embodiment, the medical device 1000 further includes a storage box 700, which is assembled at the trolley 300. Optionally, the storage box 700 is located on a side of the main unit of ventilation device 100, on which side the accommodation cavity 12 is provided.

The above are only the specific embodiments of this disclosure, the scope of protection of this disclosure is not limited to this. Any technical personnel familiar with the technical field can easily think of various equivalent modifications or replacements within the scope of disclosure in this disclosure. These modifications or replacements fall into the scope of protection of this disclosure. Therefore, the protection scope of this disclosure is limited by the claims and their equivalents.

## Claims

1. A main unit of ventilation device (100), **characterized in that**, comprising:
a housing (10), which is provided with an inner cavity (11), wherein an outer side of the housing (10) is recessed inward to form an accommodation cavity (12);
a gas path assembly (20), which comprises an oxygen control assembly (24) and an output interface (22); wherein the oxygen control assembly (24) is connected with an oxygen supply apparatus; the output interface (22) is connected with an inspiratory branch, and configured to deliver to a patient, through the inspiratory branch, a gas which is formed by mixing oxygen and air;
a pressure monitoring assembly (30), which is arranged inside the inner cavity (11), wherein the pressure monitoring assembly (30) is at least connected with the gas path assembly (20), so as to monitor a relevant parameter of an input gas and/or an output gas of the gas path assembly (20);
wherein the accommodation cavity (12) is configured to removably accommodate at least one portable medical device (200); wherein the portable medical device (200) comprises an interface side (201), which side is provided with a cable interface (202), wherein when the portable medical device (200) is accommodated inside the accommodation cavity (12), the output interface (22) and the cable interface (202) are located on different sides of the housing (10).

2. The main unit of ventilation device according to claim 1, **characterized in that**, when the portable medical device (200) is accommodated inside the accommodation cavity (12), the output interface (22) and the cable interface (202) are located on two opposite sides or two adjacent sides of the housing (10).

3. The main unit of ventilation device according to claim 2, **characterized in that**, the housing (10) comprises a front plate (A1), a back plate (A2), a top plate (A3), a bottom plate (A4), a left plate (A5) and a right plate (A6), wherein the cable interface (202) is located on a side on which side the left plate (A5) is located, and the output interface (22) is located on the right plate (A6).

4. The main unit of ventilation device according to claim 1, **characterized in that**, the gas path assembly (20) further comprises a gas output assembly (23), which is configured to receive the gas which is formed by mixing oxygen and air, wherein the gas output assembly (23) comprises the output interface (22).

5. The main unit of ventilation device according to claim 3, **characterized in that**, further comprising:
a pressure sampling interface (40), which is connected with the pressure monitoring assembly (30) and the inspiratory branch, wherein the pressure sampling interface (40) and the output interface (22) are located on a same side of the housing (10); or
a pressure sampling interface (40) and an expiratory valve (50), wherein the expiratory valve (50) is assembled at the housing (10), and configured to receive a gas exhaled by the patient, wherein the pressure sampling interface (40) is arranged inside the housing (10), and is connected with the pressure monitoring assembly (30) and the expiratory valve (50).

6. The main unit of ventilation device according to claim 3, **characterized in that**, further comprising an expiratory valve (50) that comprises a gas inlet interface (51) and a gas outlet interface (52), wherein the gas inlet interface (51) is connected with an expiratory branch, wherein the gas inlet interface (51) and the output interface (22) are located on a same side of the housing.

7. The main unit of ventilation device according to claim 6, **characterized in that**, the gas outlet interface (52) is partially located on the back plate (A2) of the housing, and the gas outlet interface (52) is partially located on the right plate (A6) of the housing.

8. The main unit of ventilation device according to claim 4, **characterized in that**, further comprising a turbine mechanism (110), wherein the gas output assembly (23) is provided with an input interface (21) and the output interface (22);
the oxygen control assembly (24) is connected with the turbine mechanism (110), and is further connected with the oxygen supply apparatus, so as to converge oxygen, which is supplied by the oxygen supply apparatus, into the turbine mechanism (110); wherein the input interface (21) is connected with the turbine mechanism (110), so as to enable a gas, which is outputted by the turbine mechanism (110), to enter into the gas output assembly (23) through the input interface (21).

9. The main unit of ventilation device according to claim 1, **characterized in that**, the housing (10) comprises a top part, a bottom part, and a side part which surrounds the top part and the bottom part; wherein the accommodation cavity (12) is formed by recessing inward the side part into an interior of the housing (10), the accommodation cavity (12) comprises a bottom wall and a side wall which surrounds the bottom wall, wherein the bottom wall is opposite to a cavity opening of the accommodation cavity (12);
the side wall is provided with a first guide structure (101) which extends along an assembling direction of the portable medical device (200); wherein the first guide structure (101) cooperates with a second guide structure (102) which is arranged at the portable medical device (200), so as to form a guide when the portable medical device (200) is assembled into the accommodation cavity (12).

10. The main unit of ventilation device according to claim 1, **characterized in that**, further comprising a turbine mechanism (110), which is arranged inside the inner cavity (11) and is connected with the gas path assembly (20); wherein the turbine mechanism (110) and the accommodation cavity (12) are arranged side by side in a height direction of the main unit of ventilation device (100), the gas path assembly (20) and the accommodation cavity (12) are arranged diagonally.

11. The main unit of ventilation device according to claim 10, **characterized in that**, further comprising an upper region (G1) and a lower region (G2), wherein the upper region (G1) comprises a first region (G11) and a second region (G12) which is arranged side by side with the first region (G11), the lower region (G2) comprises a third region (G21) and a fourth region (G22) which is arranged side by side with the third region (G21), wherein the first region (G11) and the third region (G21) are arranged side by side in the height direction of the main unit of ventilation device (100), the second region (G12) and the fourth region (G22) are arranged side by side in the height direction of the main unit of ventilation device (100);
wherein the main unit of ventilation device further comprises an expiratory valve, the housing is recessed inward to form an assembly cavity, and the expiratory valve is assembled inside the assembly cavity, which assembly is capable of being disassembled;
wherein the turbine mechanism (110) is located inside the first region (G11), the gas path assembly (20) is located inside the second region (G12), the accommodation cavity (12) is located inside the third region (G21), and the assembly cavity is located inside the fourth region (G22).

12. The main unit of ventilation device according to claim 10, **characterized in that**, an air inlet (120) is arranged at a back part of the main unit of ventilation device (100), the air inlet (120) is connected with the turbine mechanism (110), so as to enable air to enter into the turbine mechanism (110) through the air inlet (120).

13. The main unit of ventilation device according to claim 12, **characterized in that**, further comprising a bump (150), which protrudes from the back part of the housing (10).

14. The main unit of ventilation device according to claim 1, **characterized in that**, the housing (10) is provided with a gas inlet (14) and a gas outlet (15), wherein the gas inlet (14) and the gas outlet (15) are located on opposite sides or adjacent sides of the housing (10); one of the gas inlet (14) and the gas outlet (15) is adjacent to a top part of the housing (10), and the other one of the gas inlet (14) and the gas outlet (15) is adjacent to a bottom part of the housing (10);
the main unit of ventilation device (100) further comprises a gas drive assembly (160), which is arranged inside the inner cavity (11), and configured to drive a gas to enter into the inner cavity (11) from the gas inlet (14) and then exit out of the inner cavity (11) from the gas outlet (15).

15. The main unit of ventilation device according to claim 14, **characterized in that**, the accommodation cavity (12) is located on a left side or a right side of the housing (10), wherein a first hand-holding position (S1) is arranged at a top side of the accommodation cavity (12), the gas inlet (14) is arranged at the first hand-holding position (S1), and the gas outlet (15) is arranged at a back part of the housing (10).

16. The main unit of ventilation device according to claim 14, **characterized in that**, the gas drive assembly (160) comprises:
a first fan (1601), which is arranged on a back plate of the housing (10);
a second fan (1602), which is arranged at the gas outlet (15).

17. The main unit of ventilation device according to claim 14, **characterized in that**, further comprising a turbine mechanism (110), which is arranged inside the inner cavity (11) and is connected with the gas path assembly (20); wherein the housing (10) comprises a back plate (A2) and a front plate (A1) which is opposite to the back plate (A2); the back plate (A2) of the housing (10), the turbine mechanism (110), the gas path assembly (20) and the pressure monitoring assembly (30) form a first gas flow channel; the front plate (A1) of the housing (10), the turbine mechanism (110), the gas path assembly (20) and the pressure monitoring assembly (30) form a second gas flow channel.

18. The main unit of ventilation device according to claim 1, **characterized in that**, the housing (10) comprises a first side plate, a second side plate and a third side plate, wherein the first side plate, the second side plate and the third side plate are on different surfaces and are connected with one another, wherein the accommodation cavity (12) is formed by recessing inward one, two or three of the first side plate, the second side plate and the third side plate.

19. The main unit of ventilation device according to claim 3, **characterized in that**, further comprising an oxygen interface (60), which is connected with the oxygen control assembly (24) through a pipeline, wherein the oxygen interface (60) is connected with the oxygen supply apparatus to deliver oxygen, which is supplied by the oxygen supply apparatus, to the oxygen control assembly (24), wherein the oxygen interface (60) and the cable interface (202) are located on a same side of the housing (10).

20. The main unit of ventilation device according to claim 19, **characterized in that**, the accommodation cavity (12) is provided with a cavity opening, wherein the oxygen interface (60) and the cavity opening are located on a same side of the housing (10), and the accommodation cavity (12) is adjacent to the bottom plate (A4) and the back plate (A2).

21. The main unit of ventilation device according to claim 1, **characterized in that**, further comprising a first position-limiting structure (70), which is connected with the housing (10) and configured to cooperate with the portable medical device (200), so as to enable the portable medical device (200) to be stably accommodated inside the accommodation cavity (12).

22. The main unit of ventilation device according to claim 21, **characterized in that**, the first position-limiting structure (70) comprises a position-limiting member (71), which is movably connected with the housing (10), wherein the position-limiting member (71) has an open position and a close position;
when the position-limiting member (71) moves to the open position, the portable medical device (200) is capable of being freely assembled into and disassembled out of the accommodation cavity (12); when the position-limiting member (71) moves to the close position, the position-limiting member (71) abuts against the portable medical device (200), so as to limit the portable medical device (200) from being disassembled out of the accommodation cavity (12).

23. The main unit of ventilation device according to claim 22, **characterized in that**, the position-limiting member (71) is arranged at an edge of the accommodation cavity (12) and rotatably connected with the housing (10); when the position-limiting member (71) is rotated to the open position, the position-limiting member (71) is located outside a contour of the accommodation cavity (12); when the position-limiting member (71) is rotated to the close position, the position-limiting member (71) is partially located within the contour of the accommodation cavity (12), so as to limit the portable medical device (200) from being disassembled out of the accommodation cavity.

24. The main unit of ventilation device according to claim 22, **characterized in that**, an inner side wall of the accommodation cavity (12) is provided with a slide groove, an outer side surface of the housing (10) is provided with an operation port (13) which is connected with the slide groove;
wherein the first position-limiting structure (70) comprises:
the position-limiting member (71), which comprises a position-limiting part (711) and an operation part (712) which is connected with the position-limiting part (711), wherein the position-limiting part (711) penetrates into and is slidably arranged inside the slide groove, the operation part (712) penetrates into and is arranged inside the operation port (13), so as to drive the position-limiting part (711) to move to the open position by operating the operation part (712), wherein in the open position, the position-limiting part (711) is located inside the slide groove;
a first elastic member (72), which is arranged inside the slide groove and abuts against the position-limiting part (711), wherein the first elastic member (72) is configured to provide a drive force to drive the position-limiting part (711) to move to the close position; wherein in the close position, the position-limiting part (711) partially extends into the accommodation cavity, so as to limit the portable medical device (200) from being disassembled out of the accommodation cavity (12).

25. The main unit of ventilation device according to claim 21, **characterized in that**, the housing (10) comprises a top part, a bottom part, and a side part which surrounds the top part and the bottom part; wherein the accommodation cavity (12) is formed by recessing inward the side part into an interior of the housing (10), the accommodation cavity (12) comprises a bottom wall (121) and a first side wall (122) which is adjacent to the bottom wall (121), wherein the bottom wall (121) is opposite to an cavity opening of the accommodation cavity (12);
wherein the portable medical device (200) is further provided with a second position-limiting structure (203), wherein the first position-limiting structure (70) comprises:
a protrusion (73), which protrudes from the first side wall (122), and is snapped into the second position-limiting structure (203) which is arranged at the portable medical device (200), so as to limit the portable medical device (200) from being disassembled out of the accommodation cavity;
a lifting assembly (74), which is configured to lift the portable medical device (200), when subjected to an external force, so as to enable the second position-limiting structure (203) of the portable medical device (200) to be separated from the protrusion.

26. The main unit of ventilation device according to claim 25, **characterized in that**, the first position-limiting structure (70) further comprises an outer housing (75), which is provided with the protrusion (73), wherein the lifting assembly (74) comprises:
a lifting member (741), which is slidably connected with the outer housing (75) along a first direction, and configured to lift the portable medical device (200) along the first direction;
a pushing member (742), which is slidably connected with the outer housing (75) along a second direction, and configured to drive the lifting member (741) to ascend, so as to lift the portable medical device (200), wherein an angle exists between the second direction and the first direction.

27. The main unit of ventilation device according to claim 26, **characterized in that**, the lifting assembly (74) further comprises a first restoration member, which is arranged at the outer housing (75) and configured to provide a drive force to drive the lifting member (741) to descend.

28. The main unit of ventilation device according to claim 26, **characterized in that**, the lifting assembly (74) further comprises a second restoration member, which is arranged at the outer housing (75) and configured to provide a drive force to drive the pushing member (742) to restore.

29. The main unit of ventilation device according to claim 26, **characterized in that**, the first position-limiting structure (70) further comprises a pushing-out mechanism (76), which is arranged at the bottom wall (121) and configured to provide a drive force to push the portable medical device (200) out of the accommodation cavity (12).

30. The main unit of ventilation device according to claim 29, **characterized in that**, the bottom wall (121) is provided with a hole (1211);
wherein the pushing-out mechanism (76) comprises:
a pushing-out member (761), which is arranged inside the inner cavity (11) and partially extends along the hole (1211);
a second elastic member (762), which is arranged inside the hole (1211) and abuts against the pushing-out member (761), wherein the second elastic member (762) is configured to provide a drive force for the pushing-out member (761) to extend out of the hole (1211).

31. The main unit of ventilation device according to claim 25, **characterized in that**, the accommodation cavity (12) comprises the bottom wall (121), and the first side wall (122) and a second side wall (123) which are respectively arranged on opposite sides of the bottom wall (121), wherein the bottom wall (121) is opposite to the cavity opening of the accommodation cavity (12);
the first position-limiting structure (70) comprises a blocking part (77) which is arranged on the first side wall (122), the second position-limiting structure (203) comprises an elastic sheet (2031) and an abutting part (2032), wherein the elastic sheet (2031) comprises a first end and a second end which is opposite to the first end; the first end of the elastic sheet (2031) is connected with the portable medical device (200), the abutting part (2032) is connected with the elastic sheet (2031); when the portable medical device (200) is accommodated inside the accommodation cavity, the abutting part (2032) abuts against the blocking part (77) to limit the portable medical device (200) from being disassembled, wherein the abutting part (2032) is capable of being separated from the blocking part (77) by pressing the elastic sheet (2031).

32. The main unit of ventilation device according to claim 31, **characterized in that**, the first side wall (122) is provided with a first hand-holding position (S1) at an edge of the accommodation cavity (12).

33. The main unit of ventilation device according to claim 32, **characterized in that**, the second end of the elastic sheet (2031) is exposed at the first hand-holding position (S1).

34. The main unit of ventilation device according to claim 32, **characterized in that**, the second side wall (123) is provided with a second hand-holding position (S2) at an edge of the accommodation cavity (12).

35. The main unit of ventilation device according to claim 1, **characterized in that**, the accommodation cavity (12) comprises a bottom wall (121), which is opposite to a cavity opening of the accommodation cavity (12), wherein the bottom wall (121) is provided with a first interface assembly (1212), which is in electrical or wireless connection with a second interface assembly (204) which is arranged at the portable medical device (200).

36. The main unit of ventilation device according to claim 1, **characterized in that**, further comprising a handle (80), which is connected with the housing (10), wherein the handle (80) is provided with an indication structure.

37. The main unit of ventilation device according to claim 36, **characterized in that**, further comprising a front side which is provided with an operation panel, wherein the handle (80) comprises a front side surface which faces the front side of the main unit of ventilation device (100), the indication structure is arranged on the front side surface of the handle (80).

38. The main unit of ventilation device according to claim 37, **characterized in that**, the handle (80) comprises:
a cross rod (81), which is located at a top part of the housing (10) and extends along a left-right direction of the housing (10), the indication structure is arranged at the cross rod (81), wherein the cross rod (81) comprises a first end and a second end;
a first connection rod (82), which is connected with the first end of the cross rod (81) and the housing (10);
a second connection rod (83), which is connected with the second end of the cross rod (81) and the housing (10);
wherein, the cross rod (81), the first connection rod (82) and the second connection rod (83) together form a U-shaped structure.

39. The main unit of ventilation device according to claim 1, **characterized in that**, further comprising a protective member (90), which is connected with the housing (10) and protrudes from a surface of the housing (10) at a height which is greater than a height at which the portable medical device (200) protrudes from said surface of the housing (10).

40. The main unit of ventilation device according to claim 39, **characterized in that**, the housing (10) comprises a front side, a back side, a left side and a right side; wherein the left side comprises a first top edge (L1), a first bottom edge (L2), and a first side edge (L3) which is adjacent to the front side; the right side comprises a second top edge (L4), a second bottom edge (L5), and a second side edge (L6) which is adjacent to the front side; the back side comprises a third bottom edge (L7);
the protective member (90) is in a shape of an elongated strip and extends sequentially along the first top edge (L1), the first side edge (L3), the first bottom edge (L2), the third bottom edge (L7), the second bottom edge (L5), the second side edge (L6) and the second top edge (L4).

41. The main unit of ventilation device according to claim 1, **characterized in that**, the portable medical device (200) is a monitoring device.

42. The main unit of ventilation device according to claim 1, **characterized in that**, further comprising at least one portable medical device (200), wherein the portable medical device (200) is removably accommodated inside the accommodation cavity (12).

43. The main unit of ventilation device according to claim 3, **characterized in that**, further comprising a display screen (103), which is arranged at the front plate (A1), wherein the display screen (103) is at least configured to display a respiratory waveform and a monitoring parameter.

44. A main unit of ventilation device, **characterized in that**, comprising:
a housing (10), which is provided with an inner cavity (11), wherein an outer side of the housing (10) is recessed inward to form an accommodation cavity (12);
a display screen (103), which is assembled at the housing (10);
a gas path assembly (20), which is arranged inside the inner cavity (11); wherein the gas path assembly (20) comprises an oxygen control assembly (24) and an output interface (22); wherein the oxygen control assembly (24) is connected with an oxygen supply apparatus; the output interface (22) is connected with an inspiratory branch, and configured to deliver a gas to a patient, through the inspiratory branch;
a pressure monitoring assembly (30), which is arranged inside the inner cavity (11), wherein the pressure monitoring assembly (30) is at least connected with the gas path assembly (20), so as to monitor a relevant parameter of an input gas and/or an output gas of the gas path assembly (20);
wherein the accommodation cavity (12) is configured to removably accommodate at least one portable medical device (200); wherein the portable medical device (200) comprises an interface side (201), which side is provided with a cable interface (202), wherein when the portable medical device (200) is accommodated inside the accommodation cavity (12), the output interface (22) and the cable interface (202) are located on two adjacent sides of the housing (10), wherein the output interface (22) is located on a side of the housing (10) on which side the display screen (103) is arranged.

45. The main unit of ventilation device according to claim 44, **characterized in that**, the gas path assembly (20) further comprises a gas output assembly (23), which is configured to receive a gas which is formed by mixing oxygen and air, wherein the gas output assembly (23) comprises the output interface (22).

46. The main unit of ventilation device according to claim 44, **characterized in that**, further comprising an expiratory valve (50), which comprises a gas inlet interface (51), wherein the gas inlet interface (51) is connected with an expiratory branch, wherein the gas inlet interface (51) and the output interface (22) are located on a same side of the housing.

47. A main unit of ventilation device, **characterized in that**, comprising:
a housing (10), which is provided with an inner cavity (11), wherein an outer side of the housing (10) is recessed inward to form an accommodation cavity (12);
a gas path assembly (20), which is arranged inside the inner cavity (11); wherein the gas path assembly (20) comprises an input interface (21) and an output interface (22), wherein the input interface (21) is configured to input a gas which is formed by mixing oxygen and air, the output interface (22) is connected with an inspiratory branch, and configured to deliver said gas to a patient, through the inspiratory branch;
a pressure monitoring assembly (30), which is arranged inside the inner cavity (11), wherein the pressure monitoring assembly (30) is at least connected with the gas path assembly (20), so as to monitor a relevant parameter of an input gas and/or an output gas of the gas path assembly (20);
wherein the accommodation cavity (12) is configured to removably accommodate at least one portable medical device (200); wherein the accommodation cavity (12) comprises an opening for inserting the portable medical device, wherein the output interface (22) and at least a portion of the opening are located on different sides of the housing (10).

48. The main unit of ventilation device according to claim 47, **characterized in that**, the portable medical device (200) is provided with a cable interface (202), and a second interface assembly (204) which is arranged opposite to the cable interface (202); wherein the accommodation cavity (12) comprises a cavity opening and a bottom wall (121) which is opposite to the cavity opening, wherein the bottom wall (121) is provided with a first interface assembly (1212), which is in communicative connection with the second interface assembly (204).

49. A ventilation device, **characterized in that**, comprising an inspiratory branch and the main unit of ventilation device (100) according to any one of claims 1 to 45, wherein the inspiratory branch is connected with the gas path assembly (20).

50. A medical device (1000), **characterized in that**, comprising:
a trolley (300), which comprises a column (301), and a support frame (302) which is assembled at the column (301);
an infusion pump assembly (400), which is assembled at the column (301);
the main unit of ventilation device (100) according to any one of claims 1 to 48, wherein the main unit of ventilation device (100) is placed on the support frame (302).

51. The medical device according to claim 50, **characterized in that**, further comprising:
an oxygen cylinder (500), wherein the oxygen cylinder (500) is assembled at the column (302) and is arranged opposite to the infusion pump assembly (400); and/or
an infusion pole (600), wherein the infusion pole (600) is assembled at the trolley (300), and configured to hang an infusion bag; and/or
a storage box (700), wherein the storage box (700) is arranged at the trolley (300).
